# EUROPEAN PATENT SPECIFICATION

(11) **EP 3 688 087 B1**
(45) Date of publication and mention of the grant of the patent: **28.10.2026**
(21) Application number: 18796152.9
(22) Date of filing: 25.09.2018
(51) Int. Cl.: C08L 1/28

(54) **BIOMIMETIC, MOLDABLE, SELF-ASSEMBLED CELLULOSE SILICA-BASED TRIMERIC HYDROGELS AND THEIR USE AS VISCOSITY MODIFYING CARRIERS IN INDUSTRIAL APPLICATIONS**
BIOMIMETISCHE, FORMBARE, SELBSTAGGREGIERTE TRIMERISCHE HYDROGELE AUF CELLULOSE-SILIKA-BASIS UND DEREN VERWENDUNG ALS VISKOSITÄTSVERÄNDERNDE TRÄGER FÜR INDUSTRIELLE ANWENDUNGEN
HYDROGELS TRIMERIQUES BIOMIMETIQUES AUTO-AGREGES ET FORMABLES A BASE DE CELLULOSE ET SILICE ET LEUR UTILISATION COMME SUPPORTS MODIFIANT DE VISCOSITE POUR DES APPLICATIONS INDUSTRIELLES

(30) Priority: 26.09.2017 US 201715716500
(43) Date of publication of application: 05.08.2020
(73) Proprietor: The Board of Trustees of the Leland Stanford Junior University, Stanford, CA 94305-2038 (US)
(72) Inventor: APPEL, Eric A., Palo Alto, CA 94304 (US); YU, Anthony C., Stanford, CA 94305 (US)
(74) Representative: J A Kemp LLP
(86) International application number: PCT/US2018/052570
(87) International publication number: WO 2019/067406

(56) References cited:
- US-A1- 2007 001 156
- SÉVERINE ROSE ET AL: "Nanoparticle solutions as adhesives for gels and biological tissues", NATURE, vol. 505, no. 7483, 11 December 2013 (2013-12-11), London, pages 382 - 385, XP055539621, ISSN: 0028-0836, DOI: 10.1038/nature12806
- YAN BAO ET AL: "Swelling behaviors of organic/inorganic composites based on various cellulose derivatives and inorganic particles", CARBOHYDRATE POLYMERS, APPLIED SCIENCE PUBLISHERS, LTD. BARKING, GB, vol. 88, no. 2, 1 January 2012 (2012-01-01), pages 589 - 595, XP028458342, ISSN: 0144-8617, [retrieved on 20120108], DOI: 10.1016/J.CARBPOL.2012.01.003

## Description

### RELATED APPLICATION

This application claims priority and other benefits from U.S. Nonprovisional Patent Application Ser. No. 15/716,500, filed September 26, 2017, entitled "Biomimetic, moldable, self-assembled cellulose silica-based trimeric hydrogels and their use as viscosity modifying carriers in industrial applications."

### TECHNICAL FIELD OF THE INVENTION

The present invention relates to the field of self-assembled biopolymeric systems, and in particular to self-assembled cellulose silica-based biopolymeric systems that form moldable hydrogels that are useful to serve as aqueous viscosity modifying carriers in various industrial and medical applications.

### BACKGROUND

Traditional, covalently cross-linked hydrogels which comprise a class of soft materials that bind and retain large amounts of water and exhibit broadly tunable mechanical properties have limited utility because their irreversible crosslinks do not allow for stimuli-responsive aqueous viscosity modification or the ability to rearrange their shape in response to applied stress (Appel et al., 2012a).

Recent advances in supramolecular chemistry and materials science have introduced moldable polymeric systems as unique solutions to many critical industrial challenges (Appel et al., 2012a; Rodell et al., 2015). Due to the exploitation of specific and tunable non-covalent interactions, moldable polymeric systems, in contrast to covalently cross-linked hydrogels, exhibit viscous flow under shear stress (shear-thinning) and rapid recovery when the applied stress is relaxed (self-healing) which allows for precise tuning of their flow properties to meet the engineering requirements for diverse applications, including injection, pumping, or spraying.

Although many industrial applications would benefit from moldable polymeric systems that facilitate administration by flow, injection, pumping or spraying, there is a recognized shortage of moldable polymeric systems that not only allow finely tunable control over their mechanical properties, but that can also be scaled up, and be produced cost-effectively and environmentally friendly.

It would be highly desirable to rationally engineer a scalable biopolymeric system that combines readily available, inexpensive and non-toxic components in such a way that they transiently and reversibly self-assemble and so allow control and adjustment to a variety of engineering requirements. The present invention addresses this need.

### SUMMARY

Compositions and methods are provided relating to scalable, biomimetic, moldable, self-assembled biopolymeric systems that find use as aqueous viscosity modifying carriers in various industrial and medical applications including as carriers of fire retardants, fertilizers, pesticides, and pipeline cleaning agents. Primary embodiments of the invention include scalable, biomimetic, moldable, self-assembled cellulose silica-based trimeric hydrogels (CSG) formed with pairs of hydroxethyl cellulose and methyl cellulose.

In various embodiments of the invention, colloidal silica nanoparticles, referred to as CSP or CSNP herein, form moldable hydrogels by selective, pH-dependent adsorption to paired biopolymers (cellulose derivatives) via multivalent and non-covalent interactions and crosslinking. This approach affords unique control over the flow properties of the cellulose silica-based hydrogels and allows self-healing and responsiveness to external stimuli which is essential for diverse applications by means of injection, pumping, or spraying, and thus renders the gels moldable. This approach is also superior to traditional methods because it does not require complex synthetic approaches or specialized small-molecule binding partners.

The present invention also provides methods for making such carriers, and for using such carriers in technical applications as an aqueous viscosity modifying delivery platform for fire retardants, fertilizers, pesticides, pipeline cleaning agents, and more. A use of such carriers as (biomimetic) scaffolds in tissue engineering, regenerative medicine, in drug and vaccine delivery, and pharmaceutical research is contemplated as well.

The above summary is not intended to include all features and aspects of the present invention nor does it imply that the invention must include all features and aspects discussed in this summary.

### BRIEF DESCRIPTION OF THE DRAWINGS

The accompanying drawings illustrate embodiments of the invention and, together with the description, serve to explain the invention. These drawings are offered by way of illustration and not by way of limitation; it is emphasized that the various features of the drawings may not be to-scale.

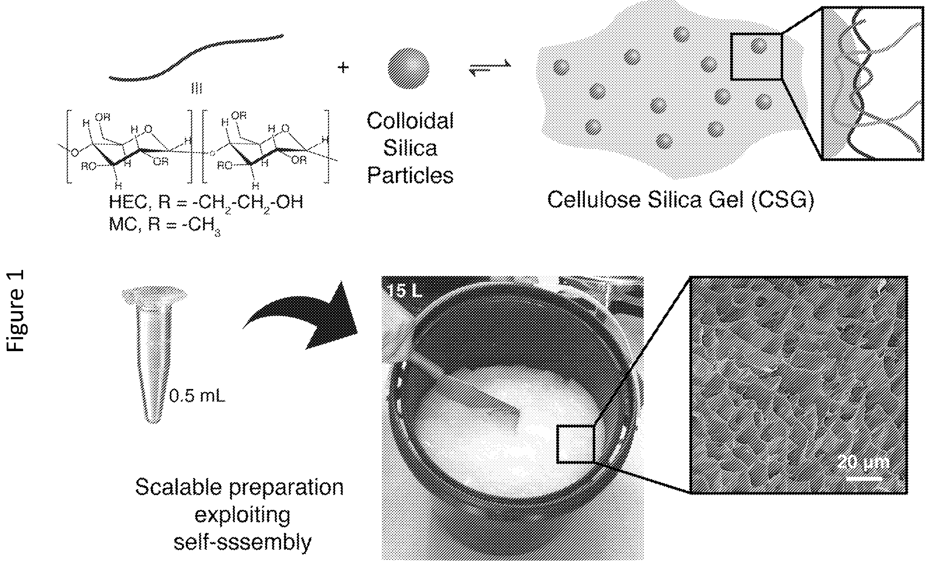

Figure 1 illustrates that biomimetic moldable cellulose silica-based trimeric hydrogels are formed through self-assembly by exploiting multivalent and non-covalent interactions between colloidal silica nanoparticles and paired biopolymers such as cellulose derivatives including hydroxyethylcellulose (HEC) and methylcellulose (MC). Here, the biopolymer chains including HEC and MC are displayed in greyscale, while colloidal silica nanoparticles are displayed in orange. Trimeric hydrogel formation by self-assembly occurs upon mixing of all three component parts which allows for facile linear scaling of the formulation from 0.5 mL to over 15 L, but the hydrogel formation only occurs if the colloidal silica particles are mixed with pairs of biopolymers.

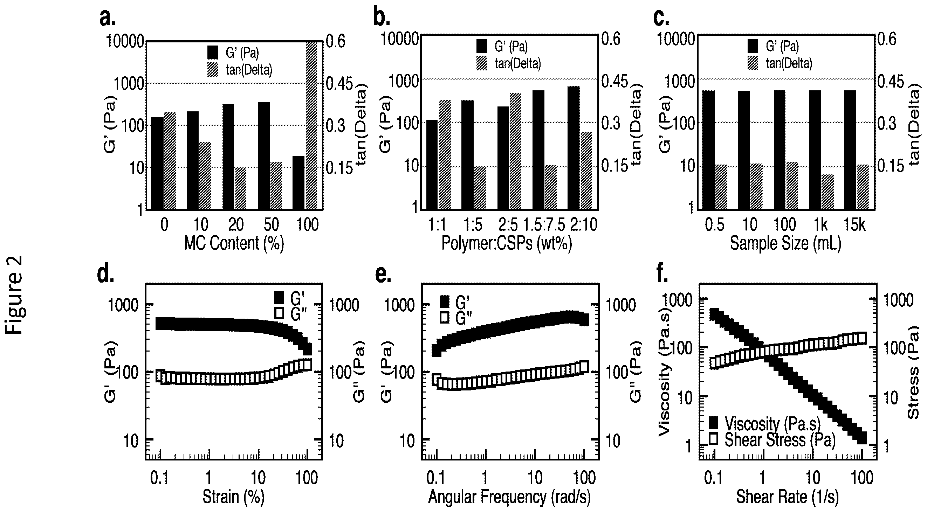

Figure 2 provides an overview over oscillatory rheological properties of various cellulose silica-based hydrogels that were prepared from (a) polymer (1 wt%) and colloidal silica (5 wt%) at various loadings (percentages) of methylcellulose (MC) in the polymer fraction ranging from 0-100% MC content, (b) hydroxyethlycellulose/methylcellulose combinations (HEC/MC) (80:20 w/w%) and colloidal silica at various ratios, and (c) HEC (1.2 wt%) / MC (0.3 wt%) / colloidal silica (7.5 wt%). All values were collected at ω = 10 rad/s, ε = 2%, 25 °C. Using a cellulose silica-based trimeric hydrogel prepared from HEC (1.2 wt%) / MC (0.3 wt%) / colloidal silica (7.5 wt%) in Fig. 2d-f, (d) strain-dependent (ω = 10 rad/s, 25°C) and (e) frequency-dependent (ε = 2%, 25 °C) oscillatory shear rheological properties were determined, and (f) steady shear rheological properties.

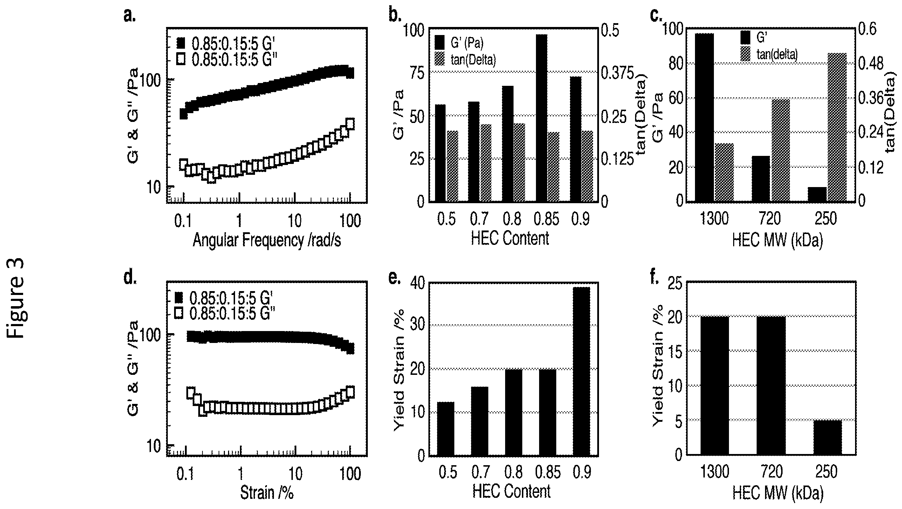

Figure 3 provides a further overview over oscillatory rheological properties obtained from various colloid silica-based trimeric hydrogels: (a) frequency-dependent oscillatory rheological properties of colloid silica-based trimeric hydrogels comprising HEC (0.85 wt%) / MC (0.15 wt%) / CSPs (5 wt%), (b) shear storage modulus and tan(delta) (G"/G') values (ω = 10 rad/s, ε = 1%) values of colloid silica-based trimeric hydrogels comprising polymer (1 wt%) / CSPs (5 wt%) with various ratios of HEC (1300 kDa) and MC as well as (c) of colloid silica-based trimeric hydrogels comprising HEC (0.85 wt%) / MC (0.15 wt%) / CSPs (5 wt%) prepared with HEC of various molecular weights. (d) Strain-dependent oscillatory rheological properties of colloid silica-based trimeric hydrogels comprising HEC/MC (1 wt%) / CSPs (5 wt%) with varying HEC:MC ratios, (e) yield strain values taken from the oscillatory strain at deviation from the linear viscoelastic regime using colloid silica-based trimeric hydrogels comprising polymer (1 wt%) / CSPs (5 wt%) with various ratios of HEC (1300 kDa) and MC, and (f) yield strain values using colloid silica-based trimeric hydrogels comprising HEC (0.85 wt%) / MC (0.15 wt%) / CSPs (5 wt%) prepared with HEC of various molecular weights.

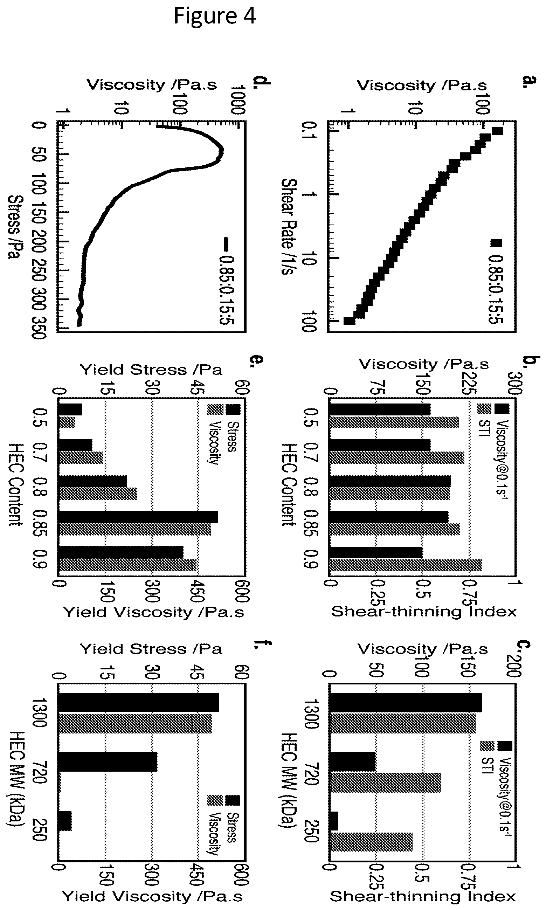

Figure 4 illustrates flow rheological properties of colloid silica-based trimeric hydrogels: (a) steady-shear flow rheological properties of colloid silica-based trimeric hydrogels comprising HEC (0.85 wt%) / MC (0.15 wt%) / CSPs (5 wt%), (b) low-shear viscosity (0.1 s⁻¹) and shear-thinning index values for colloid silica-based trimeric hydrogels comprising polymer (1 wt%) / CSPs (5 wt%) with various ratios of HEC (1300 kDa) and MC, and (c) low-shear viscosity (0.1 s⁻¹) and shear-thinning index values for colloid silica-based trimeric hydrogels comprising HEC (0.85 wt%) / MC (0.15 wt%) / CSPs (5 wt%) prepared with HEC of various molecular weights. (d) Yield-stress measurements of colloid silica-based trimeric hydrogels comprising HEC/MC (1 wt%) / CSPs (5 wt%) with varying HEC:MC ratios. (e) Yield stress and yield viscosity values for colloid silica-based trimeric hydrogels comprising polymer (1 wt%) / CSPs (5 wt%) with various ratios of HEC (1300 kDa) and MC. (f) Yield stress and yield viscosity values for colloid silica-based trimeric hydrogels comprising HEC (0.85 wt%) / MC (0.15 wt%) / CSPs (5 wt%) prepared with HEC of various molecular weights.

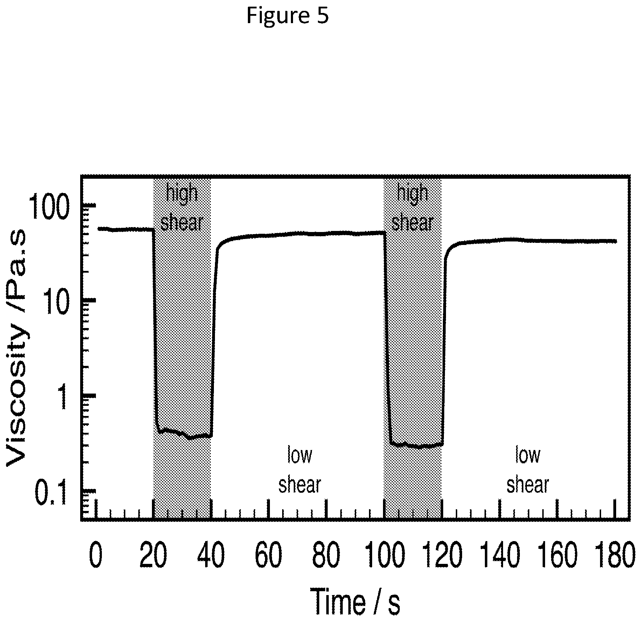

Figure 5 shows step-shear measurements of colloid silica-based trimeric hydrogels comprising HEC (0.85 wt%) / MC (0.15 wt%) / CSPs (5 wt%) over two cycles of high shear (100 s⁻¹) and low shear (0.1 s⁻¹) demonstrating rapid (less than 5s) and complete recovery of mechanical properties.

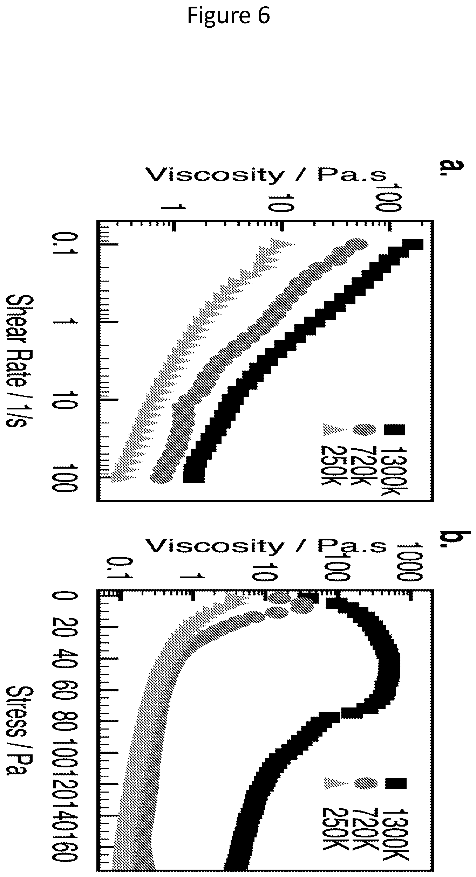

Figure 6 illustrates (a) steady-shear rheological properties and (b) yield-stress measurements of colloid silica-based trimeric hydrogels comprising HEC (0.85 wt%) / MC (0.15 wt%) / CSPs (5 wt%), demonstrating the mechanical impact of altering the HEC molecular weight from 1300 to 720 and to 250 kDa.

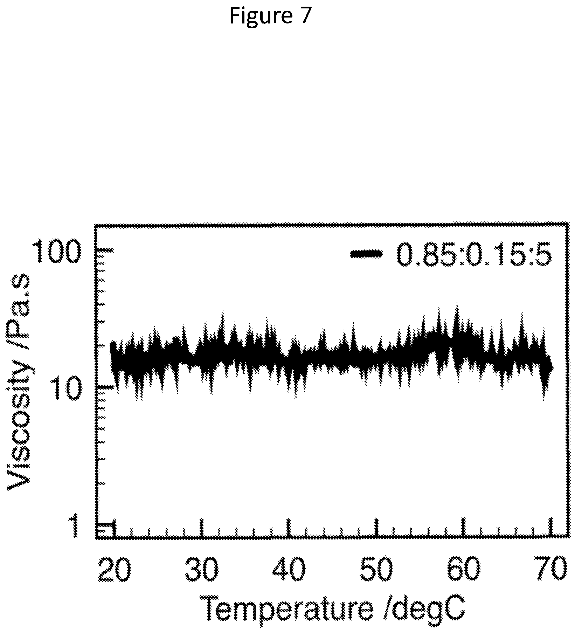

Figure 7 shows a temperature ramp of a colloid silica-based trimeric hydrogel comprising HEC (0.85 wt%) / MC (0.15 wt%) / CSPs (5 wt%) in flow (γ = 0.5 s⁻¹), demonstrating that the hydrogel's mechanical response is not affected by temperature, at least in the range of 20 to 70 degree Celsius.

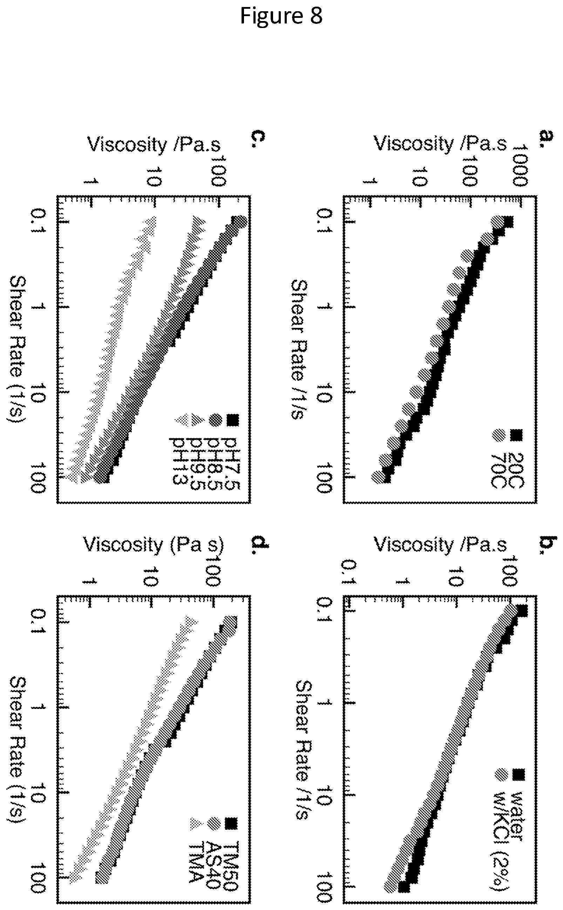

Figure 8 illustrates the stimuli-responsiveness of colloid silica-based trimeric hydrogels comprising HEC (0.85 wt%) / MC (0.15 wt%) / CSPs (5 wt%): (a) Steady-shear flow rheological properties at 20 and 70 degree Celsius. (b) Steady-shear flow rheological properties of colloid silica-based trimeric hydrogel prepared in plain water or at high salinity (2% KCl). (c) Steady-shear flow rheological properties of colloid silica-based trimeric hydrogels prepared at different pH values ranging from pH 7.5 to pH 13. (d) Steady-shear flow rheological properties of colloid silica-based trimeric hydrogels prepared with colloidal silica nanoparticles of identical size, but with different surface compositions or stabilizing counter-ion: TM50 has an anionic silica surface and sodium counter-ion, AS40 has an anionic silica surface and an ammonium counter-ion, and TMA has a cationic aluminum surface and a chloride counter-ion.

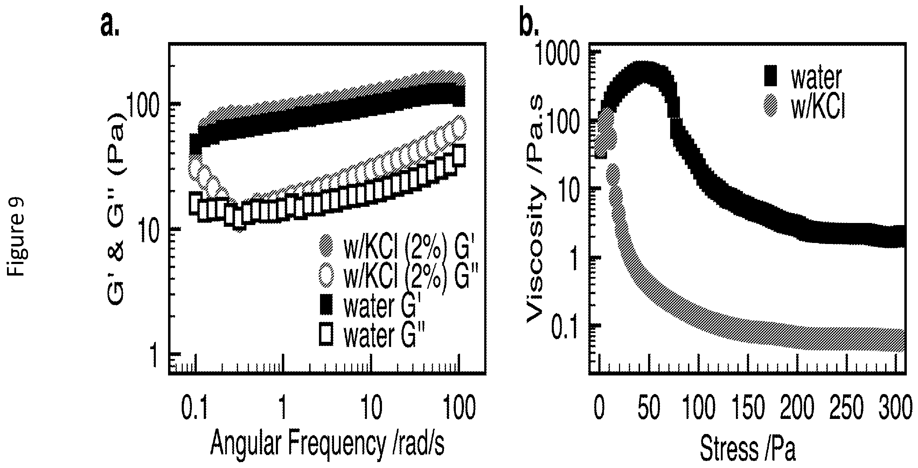

Figure 9 shows rheological properties of cellulose silica-based hydrogels prepared with KCl or water. (a) Steady-shear rheological properties of cellulose silica-based hydrogels comprising HEC (0.85 wt%) / MC (0.15 wt%) / CSPs (5 wt%) prepared at various pH values. (b) Yield-stress measurements of cellulose silica-based hydrogels comprising HEC (0.85 wt%) / MC (0.15 wt%) / CSPs (5 wt%) prepared at various pH values.

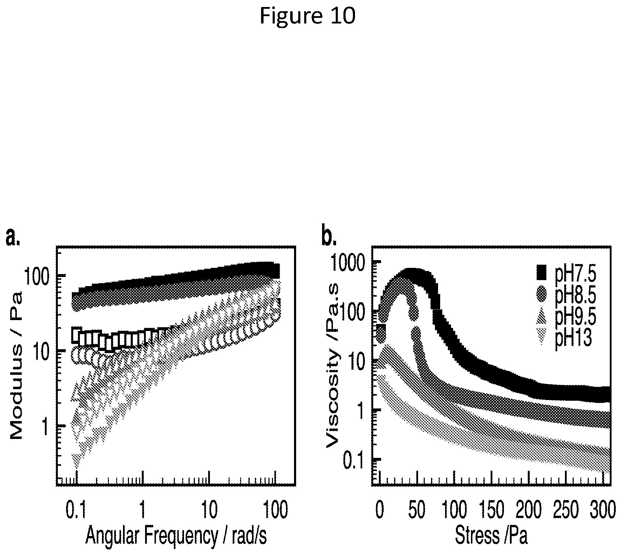

Figure 10 shows (a) steady-shear rheological properties and (b) yield-stress measurements of colloid silica-based trimeric hydrogels prepared at various pH values, comprising HEC (0.85 wt%) / MC (0.15 wt%) / CSPs (5 wt%), demonstrating the mechanical impact of altering the pH value.

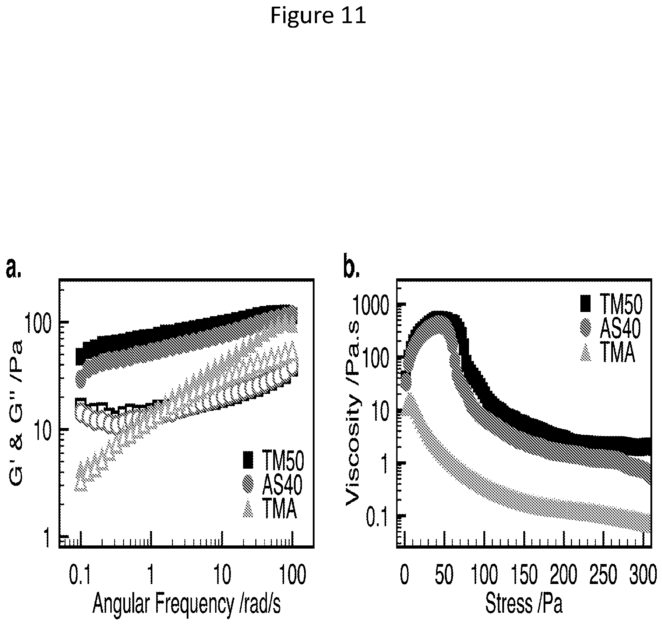

Figure 11 shows (a) steady-shear rheological properties and (b) yield-stress measurements of colloid silica-based trimeric hydrogels, comprising HEC (0.85 wt%) / MC (0.15 wt%) / CSPs (5 wt%), prepared with colloidal silica nanoparticles of identical size, but with different surface compositions or stabilizing counter-ions, demonstrating the mechanical impact of altering the colloidal silica nanoparticle. TM50 has an anionic silica surface and sodium counter-ion, AS40 has an anionic silica surface and an ammonium counter-ion, and TMA has a cationic aluminum surface and a chloride counter-ion.

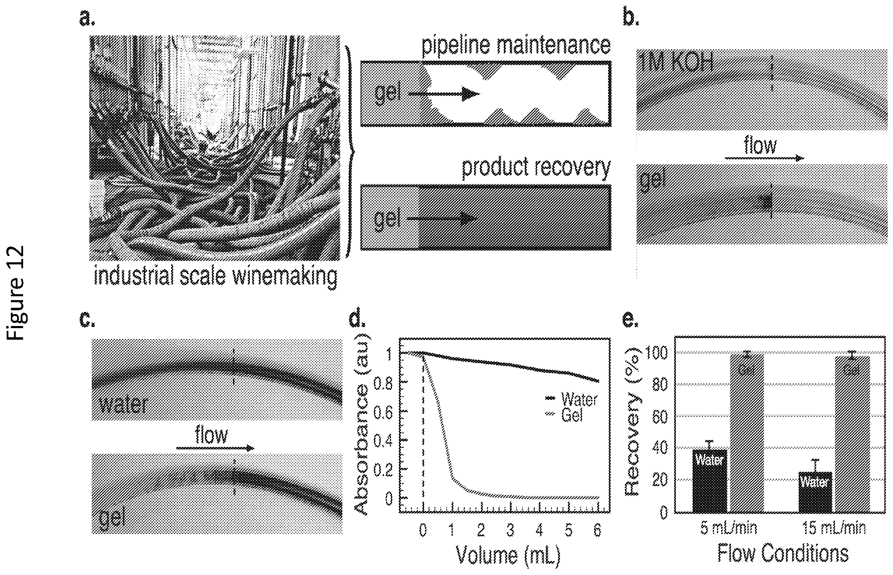

Figure 12 illustrates the principle of pipeline pigging for enhanced product recovery and more efficient pipeline maintenance. (a) Industrial wine production requires frequent pipeline cleaning and maintenance to limit contamination between products. Transfer of product through pipelines at the various stages of production typically leads to losses of approximately 2% of the total product volume. (b) Investigation of either KOH (1M; top) or cellulose silica-based hydrogel (bottom) for the removal of grape residue from aqueous solutions of Purple 8000 grape concentrate (10% v/v) allowed to foul a test pipe (*d =* 1/2", *l* = 24") for 1 h. (c) Aqueous solutions of Purple 8000 grape concentrate (10% v/v) were propelled through a test pipe (*d* = 1/2", *l* = 24") with one pipe volume (approximately 77mL) of either water (top; consistent with industry standard practice) or cellulose silica-based hydrogel (bottom). (c) By monitoring the absorbance of the Purple 8000 (λmax = 535 nm) in the eluent over time, it was possible to determine the recovery of Purple 8000 from the pipeline (using a cut-off at dilution to 80%; error bars represent one standard deviation with n=3; P<0.0001 for both groups).

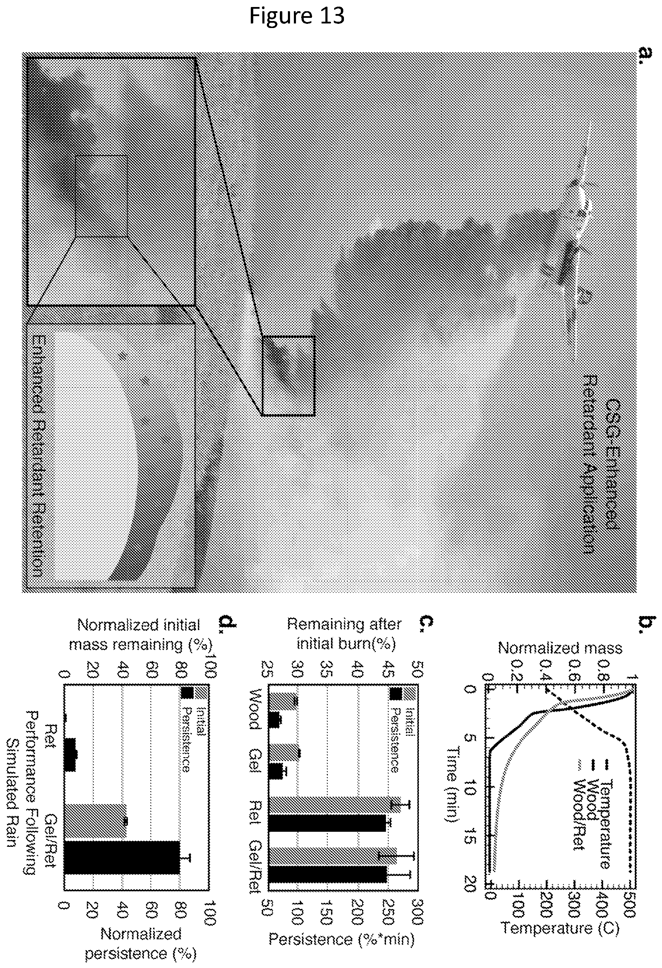

Figure 13 illustrates the utility of cellulose silica-based trimeric hydrogels as carriers of fire-retardants. (a) Select cellulose silica-based hydrogels formulated with Phos-Chek LC95A fire retardant can be sprayed in a similar fashion as standard aqueous formulations of the retardant are applied, yet provide enhanced retention at the source of the fuel. (b) Thermal gravimetric analysis (TGA) of both treated and untreated model high-surface-area to volume fuel (e.g., pinewood flour) shows that the fire retardant both decreases the dramatic initial mass loss and inhibits subsequent mass loss. (c) Quantification of the mass remaining after the initial burn and the persistence of mass over time for untreated wood, wood treated with hydrogel alone, wood treated with aqueous retardant formulation, and wood treated with retardant-containing hydrogel formulation (error bars represent one standard deviation with n=3; P<0.0001 between samples treated with and without retardant). (d) TGA analysis of wood samples treated with standard aqueous retardant formulation and fire retardant-containing hydrogel formulation following simulated rainfall. Normalization was performed on the data, whereby 0% reflects the response of wood alone and 100% reflects the response of wood freshly treated with standard aqueous retardant formulation (error bars represent one standard deviation with n=3; P<0.0001 between samples with treated with gel and those treated with standard aqueous formulations).

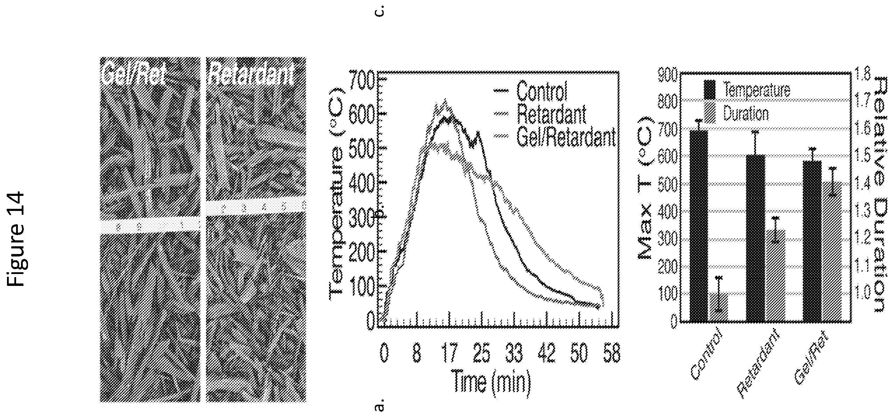

Figure 14 illustrates how fire retardant/hydrogel-based formulations and standard retardant formulations were applied using a backpack sprayer to Chamise (greasewood) chipped to a size defined as a "one-hour" fuel. The hydrogel provided enhanced surface coverage and adherence of the retardant treatment to the fuel (a). Burning the treated fuel (1 kg) in a test chamber while monitoring temperature over time with type K thermocouples demonstrated that enhanced coverage through hydrogel-based application resulted in greatly enhanced performance, indicated both by longer burn duration (i.e., slower heat release) (b, c) and lower maximum temperatures (c).

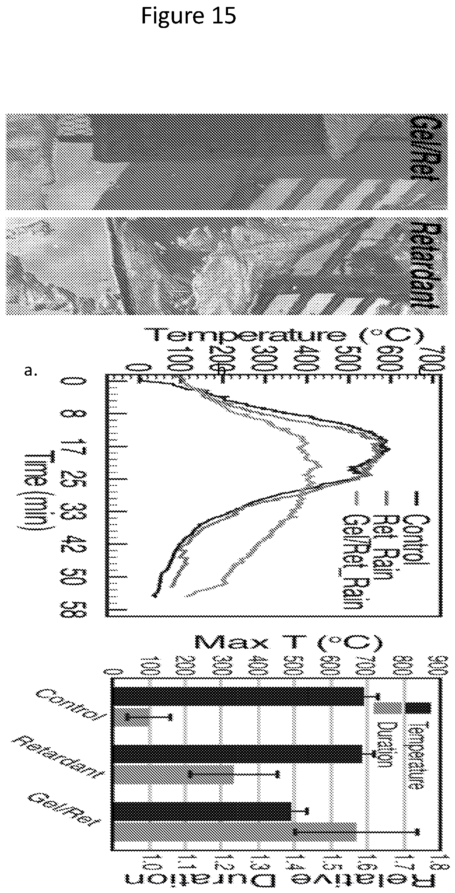

Figure 15 illustrates a simulated rain event that corresponded to approximately 0.25" of rain-fall on fuels treated with fire retardant/hydrogel-based formulations and standard retardant formulations, respectively, using a backpack sprayer. Following the rain event, the standard retardant formulations were washed away, as evidenced by the red tinted of the run-off water (a), while the hydrogel-based application resulted in complete retention of the retardant on the fuel, as subsequent burning in a test chamber showed. Burning these samples (1 kg) in a test chamber in the same manner, as described in the previous figure, indicated enhanced persistence of the fire retardant on the fuel throughout the rain event by longer burn duration (b, c) and lower maximum temperatures (c).

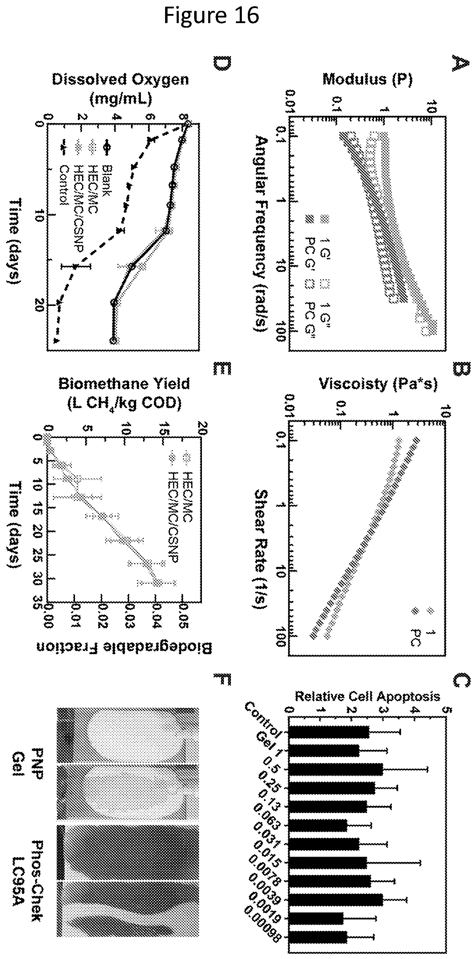

Figure 16 illustrates the viscoelasticity and shear-dependent viscosity of gel formulation 1 carrying ammonium polyphosphate (13.5 wt%) in comparison to the industry standard (PC; contains 13.5 wt% ammonium polyphosphate) based on frequency-dependent as well as flow rheometry. **(A)** Frequency-dependent rheometry shows the slight increase in modulus and viscoelasticity of gel formulation 1, which also exhibited no crossover frequency, as opposed to the commercial standard (PC). **(B)** Steady-shear viscosity measurements revealed minimal differences in flow viscosity despite the enhanced frequency-dependent rheology of gel formulation 1. **(C)** Cell cytotoxicity tests demonstrated no increase in relative cell apoptosis across all dilutions of the hydrogel. **(D)** Biological oxygen demand measurements illustrated the materials to be environmentally benign. **(E)** Biochemical methane production experiments showed slow degradation, preventing the material from degrading before the end of a fire season. **(F)** Cohesive adhesion of the hydrogels loaded with ammonium polyphosphate was visibly improved when compared to the commercial standard (PC) after 20 mL of drop-wise simulated rain.

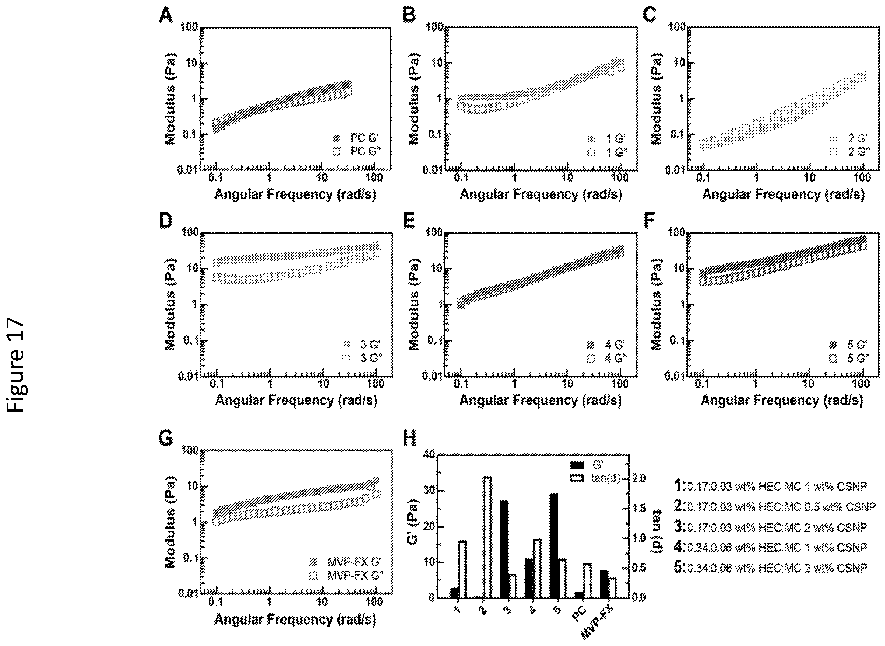

Figure 17 illustrates frequency-dependent rheological properties for each of the five tested hydrogel formulations 1, 2, 3, 4, and 5 shown in panels B, C, D, E, and F, respectively, in comparison to frequency-dependent rheological properties for the commercial standard (PC), shown in panel A. **(A)** The commercial standard (PC) exhibited a crossover frequency at ~0.4 rad/s and lower G' values than all hydrogel groups except for formulation 2. **(B)** Formulation **1** exhibited higher moduli than PC, especially at lower frequencies. **(C)** Halving the CSNP concentration seemed to reduce the crosslinking density by too much, leading to no elastic gel formation. **(D)** Doubling the CSNP concentration led to a hydrogel exhibiting higher moduli and overall more elastic behavior. **(E)** Doubling the HEC/MC concentration while keeping the CSNP concentration constant did not appreciably increase the mechanical stiffness or elasticity. **(F)** Interestingly, doubling both the HEC/MC and CSNP concentration did not significantly enhance the moduli or the relative elasticity. **(G)** MVP-FX is a fire retardant formulation that incorporates monoamonium phosphate and diammonium phosphate salts instead of ammonium polyphosphate. This lack of polyphosphates allows the MVP-FX formulation to be more rapidly mixed into solution and deployed. The formulation is gum-thickened and exhibits a modulus similar to formulation **1** and **4. (H)** Bar plots of the storage modulus, G', and tan(d) at 10 rad/s for each formulation.

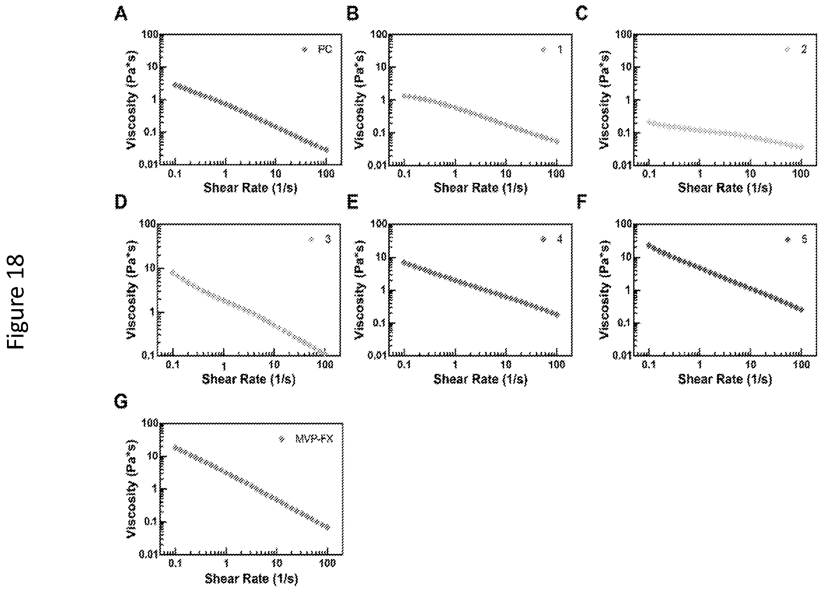

Figure 18 illustrates shear-dependent viscosity measurements for each of the tested five hydrogel formulations 1, 2, 3, 4, and 5 shown in panels B, C, D, E, and F, respectively, in comparison to shear-dependent viscosity measurements for the commercial standard (PC), shown in panel A. (A-G) Formulation **1** matched most closely to the flow profile of the commercial standard (PC). While formulation **2** exhibited much lower viscosities, formulations **3-5** had higher viscosities. These results in combination with the burn experiments suggest that viscosities that are too low may flow through the fuel instead of adhering. On the other hand, too high of a viscosity may prevent homogeneous coverage of the fuel.

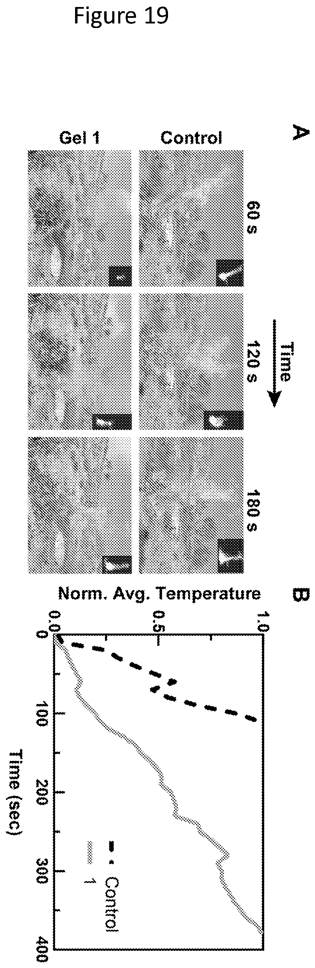

Figure 19 illustrates pilot-scale burns of chamise. **(A)** Photos of large-scale burns of chamise piles with an IR image overlay. Chamise was laid out in a 3x3 meter square, treated with PC or formulation **1** carrying ammonium polyphosphate (13.5 wt%) at CL3, dried, rained on (0.5"), dried, piled up, and then burned. An untreated starter bundle was placed at the base of the pile and used to start the fire. **(B)** The temperature-time curves were integrated and normalized to the plateau burn temperature of the untreated control pile.

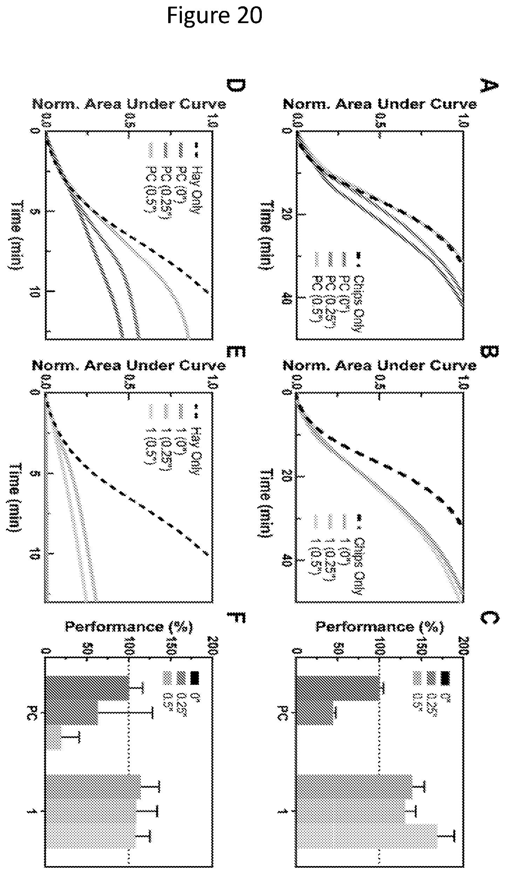

Figure 20 illustrates lab-scale burn experiments of chamise chips and hay. **(A, B)** Normalized area under the temperature versus time curves of the burn experiments on chamise chips treated with PC or formulation **1** carrying ammonium polyphosphate (13.5 wt%). Chamise chips treated with formulation **1** exhibited constant performance even up to 0.5" of rain. **(C)** Normalized performance of each treatment quantifies these results, showing that at 0.5" rain, PC has 0% performance, while all tests using formulation **1** display performance above 100%. 100% performance was defined as the total area under the temperature-time curve of PC treated hay with no rain. **(D, E)** Normalized area under the temperature versus time curves of the burn experiments on hay treated with PC or formulation **1. (F)** Normalized performance of each treatment illustrates the performance decrease of PC with rain and, in comparison, the stable performance of formulation **1.** 100% performance was defined as the time to reach 150°C for PC treated chamise chips with no rain.

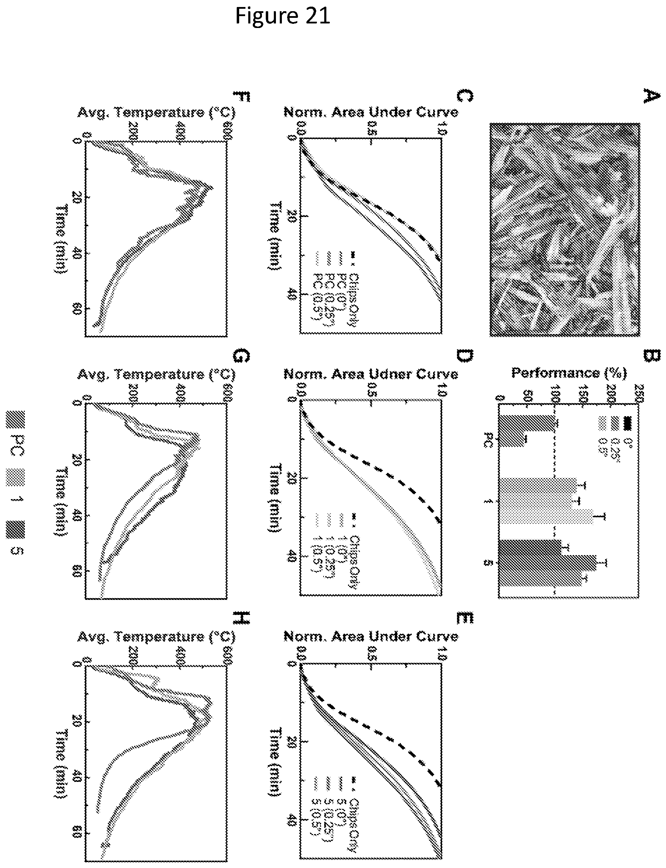

Figure 21 illustrates laboratory-scale chamise chip burn data following treatment with gel formulation 1 or gel formulation 5, both carrying ammonium polyphosphate (13.5 wt%), in comparison to the commercial standard PC. **(A)** Photo of the chamise chips treated at coverage level 1 (CL1 ) of a gel formulation. **(B)** Performance values plotted for each laboratory-scale burn experiment revealed that PC treatments were no longer effective at 0.5" of rain, while both gel treatments (1 and 5) performed above 100% at all rain levels. Performance was normalized to the total area under the curve of untreated chamise chips. **(C)** Plots of normalized area under the temperature vs time curves illustrate the enhanced performance and retention of the gel formulations. **(D)** Plots of the average temperature of the two thermocouples vs time for each treatment.

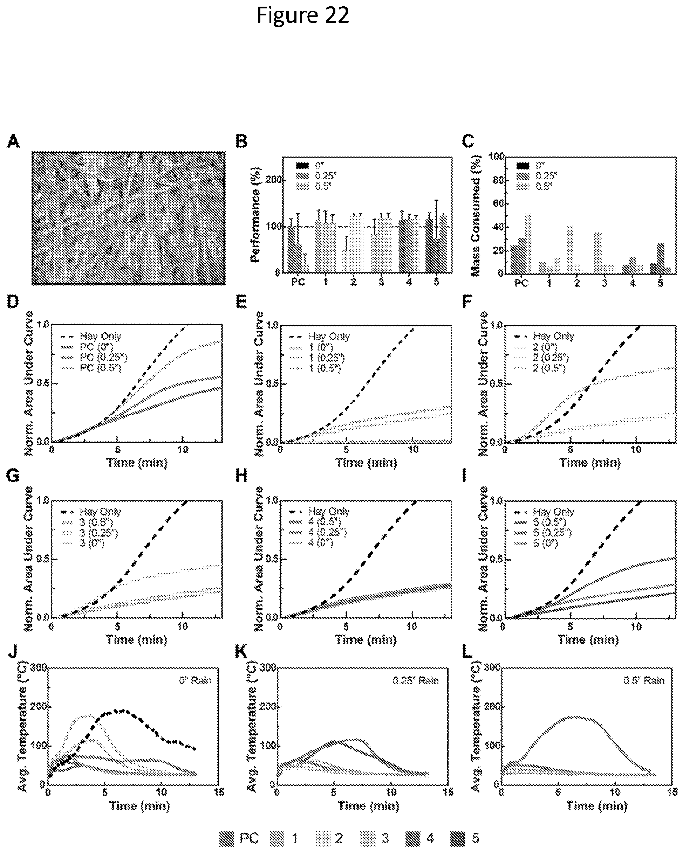

Figure 22 illustrates laboratory-scale hay burn data following treatment with gel formulations 1, 2, 3, 4 or 5, each carrying ammonium polyphosphate (13.5 wt%), in comparison to the commercial standard PC. **(A)** Photo of the hay sprayed with CL1 of a gel formulation. **(B)** Performance of each treatment was measured as the time to reach 150°C. PC treatments showed minimal efficacy after 0.5" of rain. **(C)** The mass after each burn was measured and plotted, demonstrating a significant decrease in the total mass burned for each gel formulation treatment. Markedly, gel formulations 2 and 3 exhibited a decrease in mass consumed after being rained on, possibly due to spreading of the gel formulation due to rain. **(D)** Plots of normalized area under the temperature vs time curves for each formulation compared to the untreated hay. **(E)** Plots of the average temperature vs time of each treatment after 0, 0.25, or 0.5" of simulated rain.

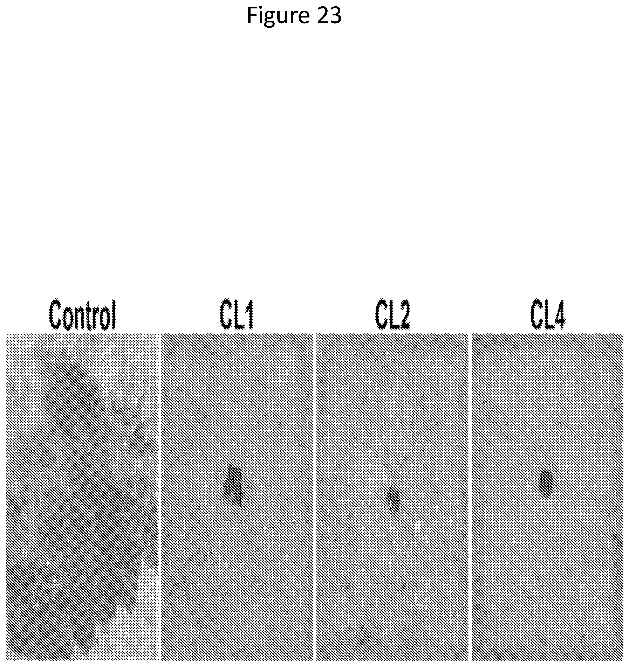

Figure 23 illustrates burn results following direct application of gel formulation 1 carrying ammonium polyphosphate (13.5 wt%) without rain showing, from left to right, overhead images of 3x3 meter plots untreated, treated with gel formulation 1 at coverage levels 1 (CL1), 2 (CL2) , or 4 (CL4). These burns were performed after direct application and no rain was simulated. Results indicate that, without rain, a CL1 treatment is sufficient to preclude fire spreading.

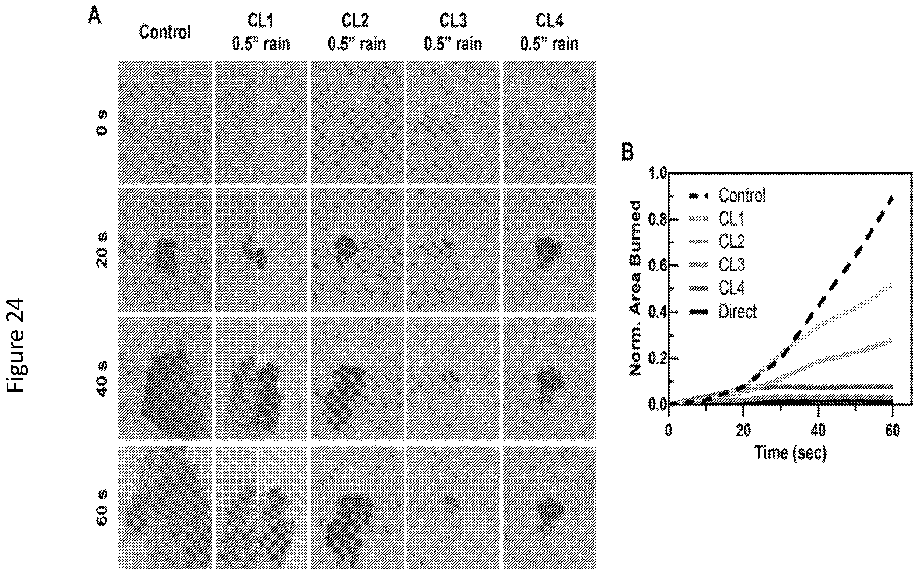

Figure 24 illustrates the time course of mowed grass burn experiments following application of gel formulation 1 carrying ammonium polyphosphate (13.5 wt%), and subsequent exposure to simulated rain. **(A)** Overhead time course images of 3x3 meter plots either untreated, or treated with different coverage levels 1, 2, 3, or 4, and subsequently exposed to 0.5" of simulated rain. Burns were performed ~1 week after application of gel formulations, exposing the sites to the environment as they dry. **(B)** The normalized area burned was quantified and demonstrated that coverage level 3 or above was sufficient to completely preclude spreading of the fire.

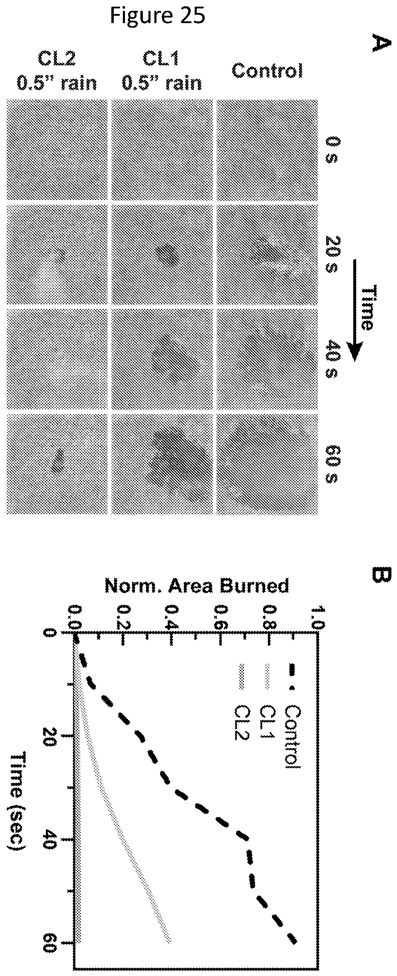

Figure 25 illustrates the time course of standing grass burn experiments following application of gel formulation **1** carrying ammonium polyphosphate (13.5 wt%) at coverage level 1 (CL1) or coverage level 2 (CL2), and subsequent exposure to simulated rain. **(A)** Overhead time course images of 3x3 meter plots either untreated or treated with different coverage levels of **1** and exposed to 0.5 inches of rain. Burns were performed ~1 week after application of gel formulations, exposing the sites to the environment as they dry. **(B)** The normalized area burned demonstrates that coverage level 2 is sufficient to preclude spreading of the fire.

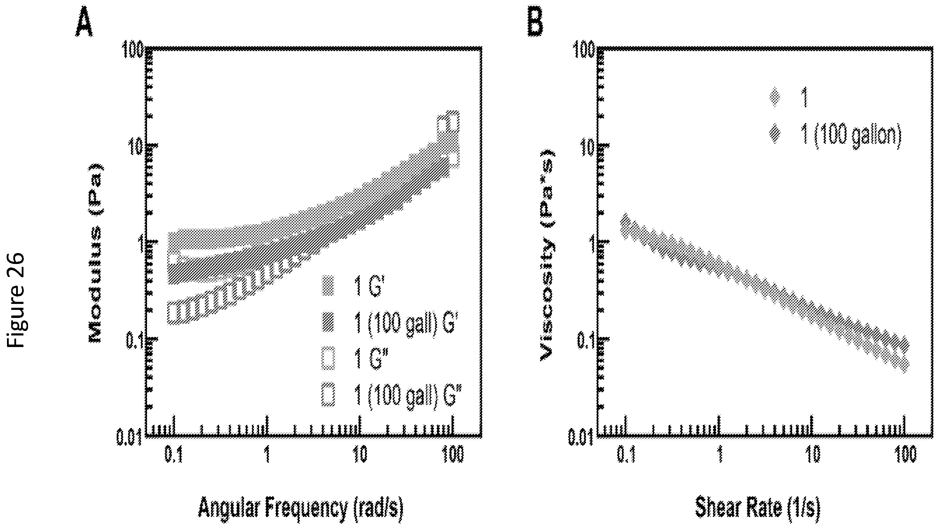

Figure 26 illustrates rheometry measurements of gel formulation 1 carrying ammonium polyphosphate (13.5 wt%) at lab-scale and at a large, 100-gallon scale. Formulation **1** produced at a 100-gallon scale did not significantly change the modulus, elasticity, or flow properties. These result demonstrate the reliability of scale-up as well as the simplicity in preparing the material. Essentially, materials components are placed in a single chamber and mixed.

### DETAILED DESCRIPTION

Compositions and methods are provided relating to scalable, biomimetic, moldable, self-assembled biopolymeric systems that find use as aqueous viscosity modifying carriers in various industrial and medical applications including as carriers of fire retardants, fertilizers, pesticides, and pipeline cleaning agents. Primary embodiments of the invention include scalable, biomimetic, moldable, self-assembled cellulose silica-based trimeric hydrogels formed through multivalent and non-covalent interactions with pairs of hydroxyethyl cellulose and methyl cellulose.

Scalable and moldable cellulose silica-based trimeric hydrogels can be prepared from environmentally safe, cost-effective and renewable polysaccharide starting materials and, therefore, present a unique solution to important industrial and environmental challenges that so far remained unsolved because of currently used materials' lack of scalability, lack of required mechanical properties, lack of environmental degradability, poor biocompatibility and unsustainable consumption of water.

In specific embodiments of the present invention, cellulose silica-based trimeric hydrogels find use in pipeline maintenance in the food and beverage manufacturing industry, as carriers of fire retardants, fertilizers, pesticides and more.

Before describing these specific embodiments of the invention, it will be helpful to set forth definitions that are used in describing the present invention.

### 1. DEFINITIONS

Unless defined otherwise, all technical and scientific terms used herein have the same meaning as commonly understood by a person of ordinary skill in the art to which this invention belongs. The following definitions are intended to also include their various grammatical forms, where applicable. As used herein, the singular forms "a" and "the" include plural referents, unless the context clearly dictates otherwise.

The term "colloidal silica," as used herein, refers to silicon dioxide and, in particular, to synthetic silica including pyrogenic silica and precipitated silica, in crystalline as well as non-crystalline (amorphous) form. Colloidal silica includes colloidal silica particles and colloidal silica nanoparticles.

The term "hydrogel," as used herein, refers to an aqueous polymeric system that is formed by multivalent and non-covalent interactions and crosslinking of polymerizing components, and is void of covalent interactions and crosslinks.

The terms "trimer" and "trimeric," as used herein, relate to the formation of a polymeric system, in particular a hydrogel, from a three-components set-up. Thus, a trimeric hydrogel represents a three-component hydrogel. In a cellulose silica-based trimeric hydrogel, one component is colloidal silica and the other two components are pairs of cellulose derivatives that only form a hydrogel with colloidal silica when both components of the cellulose derivatives pair are present, but that do not form a hydrogel with colloidal silica if only one of the cellulose derivatives pair is present.

The term "shear-thinning," as used herein, relates to the process of a hydrogel thinning under the application of tension to the point where the hydrogel starts to flow.

The term "self-healing," as used herein, relates to the process of a flowing hydrogel to reforming to a non-flowing hydrogel once the applied tension is relaxed or removed.

The term "moldable hydrogel," as used herein, relates to a hydrogel that thins upon the application of tension and rapidly self-heals once the tension is relaxed or removed.

The term "biodegradable," as used herein, relates to materials that decompose under natural conditions and are capable of being decomposed by living organisms.

The term "colloidal silica," as used herein, relates to a stable dispersion of silica particles, typically ranging from about 1 to 100 nm.

The term "viscosity modifier" or "viscosity modification," as used herein, relates to a change of flowability that is obtained by the addition of a hydrogel, as described herein.

The term "biomimetic scaffold," as used herein, relates to a three-dimensional matrix that is derived from natural body tissues or synthetic materials and that supports metabolic activities, provides biomechanical strength for tissue reconstruction in humans as well as animals, and permits typical cell type interactions in tissues. Such scaffolds can include agents such as growth factors, e.g. TGF-beta, VEGF, PDGF, BMPs, NGFs, small molecule drugs, proteins, peptides, and other additives, to enhance stability of the scaffold and to aid tissue regeneration.

The term "coverage level," as used herein defines the density of a fire retardant per a defined area. The standard levels range from 1-7 representing the number of gallons of a fire retardant or of a carrier carrying a fire retardant per a 100 square foot area. Thus, coverage level 1 (CL1) means that 1 gallon of a fire retardant or 1 gallon of a carrier carrying a fire retardant is applied per a 100 square foot area, CL2 means that 2 gallons of a fire retardant or 2 gallons of a carrier carrying a fire retardant are applied per a 100 square foot area, CL3 means that 3 gallons of a fire retardant or 3 gallons of a carrier carrying a fire retardant are applied per a 100 square foot area, and so forth.

The term "cosmetic agent," as used herein, relates to molecules and articles that are applied to the human face or body for skin cleansing or beautifying.

The term "small molecule," as used herein, relates to molecules with a molecular weight of less than 1000 daltons and having biological or pharmacological activity.

The terms "pharmacologically active/activity" and "biologically active/activity," as used herein, relate to an effect that is useful in the treatment or diagnosis of a disease or disorder or that affects the function of the body of a human or an animal.

### 2. POLYMERIC SYSTEMS

In one aspect, the present invention is directed to compositions relating to scalable, biomimetic, moldable, self-assembled biopolymeric systems that find use as cost-effective, renewable and biodegradable aqueous viscosity modifying carriers. Primary embodiments of the invention include scalable, biomimetic, moldable, self-assembled cellulose silica-based trimeric hydrogels formed by combining colloidal silica nanoparticles with pairs of polysaccharides which are produced from renewable, environmentally safe and cost-effective starting materials and which are suitable to a range of industrial applications. These compositions promise broad utility as inert and safe carriers for a variety of cargo. In exemplary embodiments, the applicability of these compositions is demonstrated as carriers for pipeline maintenance in the food and beverage industries, and as carriers of fire retardants for effective and affordable wildland fire management.

Moldable cellulose silica-based hydrogels are formed and maintained through specific and tunable multivalent and non-covalent interactions and crosslinking as evidenced by their ability to exhibit viscous flow under shear stress (shear-thinning) and rapid recovery when the applied stress is relaxed (self-healing). In contrast to traditional chemically cross-linked materials, such multivalent and non-covalent interactions and crosslinking allow for precise tuning of flow properties to meet the engineering requirements for diverse applications, including injection, pumping, or spraying.

Select cellulose derivatives have been reported to adsorb onto colloidal silica particles (Kapsabelis and Prestidge, 2000). The complementary affinity between polymers and silicates emulates natural systems and has been utilized to fabricate biomimetic hydrogels of incredible strength (Wang et al., 2010; Tamesue et al., 2013).

### Polysaccharides suitable for forming trimeric hydrogels with colloidal silica nanoparticles

Polysaccharides suitable for forming trimeric hydrogels with colloidal silica nanoparticles must be capable of selectively adsorb to colloidal silica particles via multilateral and non-covalent interactions.

Naturally occurring polysaccharides are polymers of carbohydrates such as starch, glycogen, alginate, agarose, cellulose whose properties depend on their chemical structure, functional groups, linkage, conformation, and more. Ring-opening polymerization, enzyme-catalyzed polymerization and other methods of polymerization have made possible the production of synthetic polysaccharides. Both naturally occurring and synthetic polysaccharides are contemplated as being suitable for use in the present invention, particularly derivatives of cellulose.

Chemically modified derivatives of cellulose are widely used in the industrial sector and can be obtained, for example, by esterification (acetylation, sulphation), carbanilation, and etherification of cellulose. Exemplary cellulose derivatives include cellulose acetate, cellulose nitrate, cellulose xanthate, carboxymethylcellulose, methylcellulose, ethylcellulose, hydroxyethylcellulose.

Commercially available polysaccharide compounds, including cellulose derivatives, were screened in a concentration of 1 % mass/volume (1 wt%) for their ability to form hydrogels with colloidal silica particles (*D*_{H} ~15 nm) in concentrations of 1-10 wt.% under ambient conditions.

Among the cellulose derivatives, hydroxyethylcellulose (HEC) and methylcellulose (MC) were identified to rapidly form robust trimeric hydrogels when mixed with colloidal silica particles. In each case, hydrogel formation required the presence of colloidal silica particles and both (paired) cellulose derivatives, as solutions of colloidal silica particles (1-10 wt%) and cellulose derivatives (1 wt%) alone were each low viscosity liquids.

In various embodiments of the invention, pairs of cellulose derivatives were utilized to form cellulose silica-based trimeric hydrogels, whereby various combinations of the pair hydroxyethylcellulose (HEC) and methylcellulose (MC) gave the best results.

### Colloidal silica particles suitable for hydrogel formation with pairs of cellulose derivatives by way of multivalent and non-covalent interactions and crosslinking

In various embodiments of the invention, colloidal silica nanoparticles form moldable hydrogels by selective, pH-dependent adsorption to paired cellulose derivatives via multivalent and non-covalent interactions and crosslinking. This approach affords unique control over the flow properties of the cellulose silica-based hydrogels and allows self-healing and responsiveness to external stimuli which is essential for diverse applications by means of injection, pumping, or spraying; this approach is also superior to traditional methods because it does not require complex synthetic approaches or specialized small-molecule binding partners.

Colloidal silica nanoparticles suitable for forming trimeric hydrogels with pairs of cellulose derivatives require a particular surface chemistry meaning that they have a silica surface that substantially (at least 80%) or exclusively consists of silanol (SiOH) groups and is negatively charged in a pH dependent manner.

### 3. METHODS OF USE

In another aspect, the invention provides methods for making such compositions, and for using them as carriers in in various industrial, technical and medical applications as an aqueous viscosity modifying delivery platform for fire retardants, fertilizers, pesticides, pipeline cleaning agents, and more. A use of such carriers as biomimetic scaffolds in tissue engineering, regenerative medicine, in drug and vaccine delivery, and pharmaceutical research is contemplated as well.

### Fire retardants

Fire retardant agents or fire retardants are substances that slow or stop the spread of fire. Phos-Check fire retardants, some of which were used in the experiments described herein, are mixed with water and applied to homes, trees and vegetation when there is a risk of a wild fire in the area. Fire retardants usually include phosphates such as mono-ammonium or diammonium phospate, ammonium polyphosphate, or sulfates such as diammonium sulfate.

### Chemical Treatment With Fertilizers

Fertilizers support and enhance the growth of plants and are commonly used in liquid or solid form for growing crops. A typical fertilizer contains nitrogen, phosphorus, or potassium. Many of the above mentioned fire retardant phosphates and sulfates double as fertilizers. Since excess fertilization is counterproductive, fertilizer must be applied carefully and efficiently.

### Chemical Treatment with Pesticides

Pesticides are chemical or biological agents that regulate the growth of weeds (herbicides) and pests (pesticides). Since pesticides can have toxic effects in humans and animals when ingested accidentally, pesticides must be applied in a controlled fashion.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible. In the following, experimental procedures and examples will be described to illustrate parts of the invention.

### EXPERIMENTAL PROCEDURES

The following methods and materials were used in the examples that are described further below.

### Cellulose silica-based trimer hydrogel preparations

Cellulose silica-based trimer hydrogels were prepared by first dissolving hydroxyethylcellulose (HEC; *M*ᵥ ~ 1300 or 720 kDa; Sigma) and/or methylcellulose (MC; *M*ᵥ ~ 90 or 60 kDa; Sigma) in water (30 mg/mL) with stirring and mild heating. Colloidal silica particles (CSPs; Ludox TM-50; *D*_{H} ~ 15nm; 50% w/w; Sigma) were diluted to 15 wt% solutions. HEC/MC polymer solution (150 µL) and CSP solution (300 µL) were then added together and mixed well by vortex (some samples were mildly centrifuged to remove bubbles arising from mixing). Large-volume preparation of cellulose silica-based trimer hydrogels used the same ratios scaled linearly, and mixing was performed with an impeller mixer.

### Cellulose silica-based hydrogel characterization

Rheological characterization was performed using a TA Instruments AR-G2 controlled-stress rheometer fitted with a Peltier stage. All measurements were performed using a 25 mm plate geometry and analyzed using TA Instruments TA Orchestrator software. SEM images where acquired using a FEI XL30 Sirion microscope using a beam voltage of 5 kV. Lyophilized samples were pressed onto carbon paint and sputter coated with Au/Pd (60:40) prior to imaging.

### Pipeline pigging experiments

Two experiments were designed to investigate the performance of the cellulose silica-based trimer hydrogels in pigging applications. For the first experiment, to simulate wine product movement, an aqueous solution of Purple 8000 grape concentrate (10% v/v) was pumped into a test pipe (*d =* 1/2", *l* = 24"), followed immediately by either hydrogel (prepared as mentioned above) or water (control experiment consistent with industry practice). The absorbance (λₘₐₓ = 535 nm) of the eluent was monitored over time at varying flow rates. Analysis of the proportion of aqueous Purple 8000 recovered from the pipe was determined utilizing a cut-off at 80% dilution. For the second experiment, to simulate pipeline cleaning, Purple 8000 solutions were pumped into the test pipe and allowed to settle for 1h before draining. Following this, either hydrogel (prepared as mentioned above) or water was pumped through the pipe at varying flow rates for cleaning. Analysis of cleaning potential was determined by monitoring absorbance (λₘₐₓ = 535 nm) of the eluent over time. All experiments were performed in triplicate.

### Fire prevention experiments

Two experiments were designed to investigate the performance of the cellulose silica-based trimer hydrogels as carriers of fire retardants in fire prevention applications. Initially, wood flour (System Three, Inc.) was dried at 120 °C under vacuum for 24h. Dry wood flour (100 mg) was mixed with each treatment (20 mg), including water (control), gel, fire retardant solution, and gel containing fire retardant. Each treatment group was dried at 120 °C under vacuum for 24h prior to analysis. Utilizing thermal gravimetric analysis (TGA), samples (~20 mg) were heated at a rate of 50 °C/min to 500 °C, and then maintained at 500 °C for 15 min while monitoring weight loss. Subsequently, each treatment group was subjected to simulated rain, whereby samples were washed with water with vigorous shaking (1 mL for 10 s), isolated by filtration, and dried at 120 °C under vacuum for 24h prior to TGA analysis. All experiments were performed in triplicate.

### EXAMPLES

The following examples are put forth so as to provide those of ordinary skill in the art with a complete disclosure and description of how to make and use the present invention; they are not intended to limit the scope of what the inventors regard as their invention. Unless indicated otherwise, part are parts by weight, molecular weight is average molecular weight, temperature is in degrees Centigrade, and pressure is at or near atmospheric.

### EXAMPLE 1: BIOMIMETIC CELLULOSE SILICA-BASED HYDROGEL FORMATION [AND CHARACTERIZATION]

Within the field of self-assembly, interactions between polymers and nano-sized particles have arisen as a simple route to assemble tunable and self-healing polymeric materials without the need for complex synthetic approaches or specialized small-molecule binding partners (Rose et al., 2014; Appel et al., 2015a; Appel et al., 2015b). The complementary affinity between polymers (molecular binders) and hard nanoparticles (clay nanosheets/silicates) emulates natural systems and has been utilized to fabricate biomimetic hydrogels of incredible strength (Wang et al., 2010; Tamesue et al., 2013).

Herein, we report the preparation and application of biomimetic cellulose silica-based hydrogels driven by non-covalent interactions between renewable and environmentally benign polysaccharides and colloidal silica particles, as shown in Figure 1. Polysaccharides provide an attractive starting material on account of their availability from renewable resources, aqueous solubility, high molecular weight, diverse chemical functionality, low cost, environmental-degradability, biocompatibility, and large scale production (Rose et al., 2014). Moreover, colloidal silica particles are produced on large scale and utilized across many fields and technologies ranging from personal care products to fining agents in industrial beer and wine production, and yet many fundamental questions regarding the formation and behavior of colloidal silica composites and systems are still not fully addressed (Bergna and Roberts, 2005).

### CONDITIONS FOR HYDROGEL FORMATION FROM POLYMER AND COLLOIDAL SILICA NANOPARTICLES (CSP or CSNP)

Efficient cross-linking in polymer-nanoparticle hydrogels necessitates three criteria: (i) a strong affinity between the nanoparticles and the polymer chains, *i.e.,* the free energy gain (ε) resulting from adsorption of a polymer chain to the surface of a nanoparticle should be greater than or comparable to the thermal energy (*k*_{B}*T*); (ii) the nanoparticle diameter should be comparable to, or less than, the persistence length (*l*_{P}) of the polymer strands (required to favor polymer bridging of multiple nanoparticles over polymer wrapping around individual particles); and (iii) the number density of polymers and particles must allow for an average at least two interactions per particle and polymer to comprise a cross-linked network (Appel et al., 2015a). When these criteria are met, hydrogel formation is favored over the alternative conformations.

### MATERIALS

Commercially available polysaccharide compounds in a concentration of 1 % mass/volume (1 wt%) were screened for their ability to form hydrogels with colloidal silica particles (*D*_{H} ~15 nm) in concentrations of 1-10 wt% under ambient conditions. Through qualitative screens hydroxyethylcellulose (HEC) and methylcellulose (MC) were identified to rapidly form robust hydrogels when mixed with colloidal silica particles, as shown Table 1 with a '+' in the performance column (far right). In each case, hydrogel formation required the presence of both colloidal silica particles and cellulose derivatives, as solutions of colloidal silica particles (1-10 wt%) and cellulose derivatives (1 wt%) alone are each low viscosity liquids.

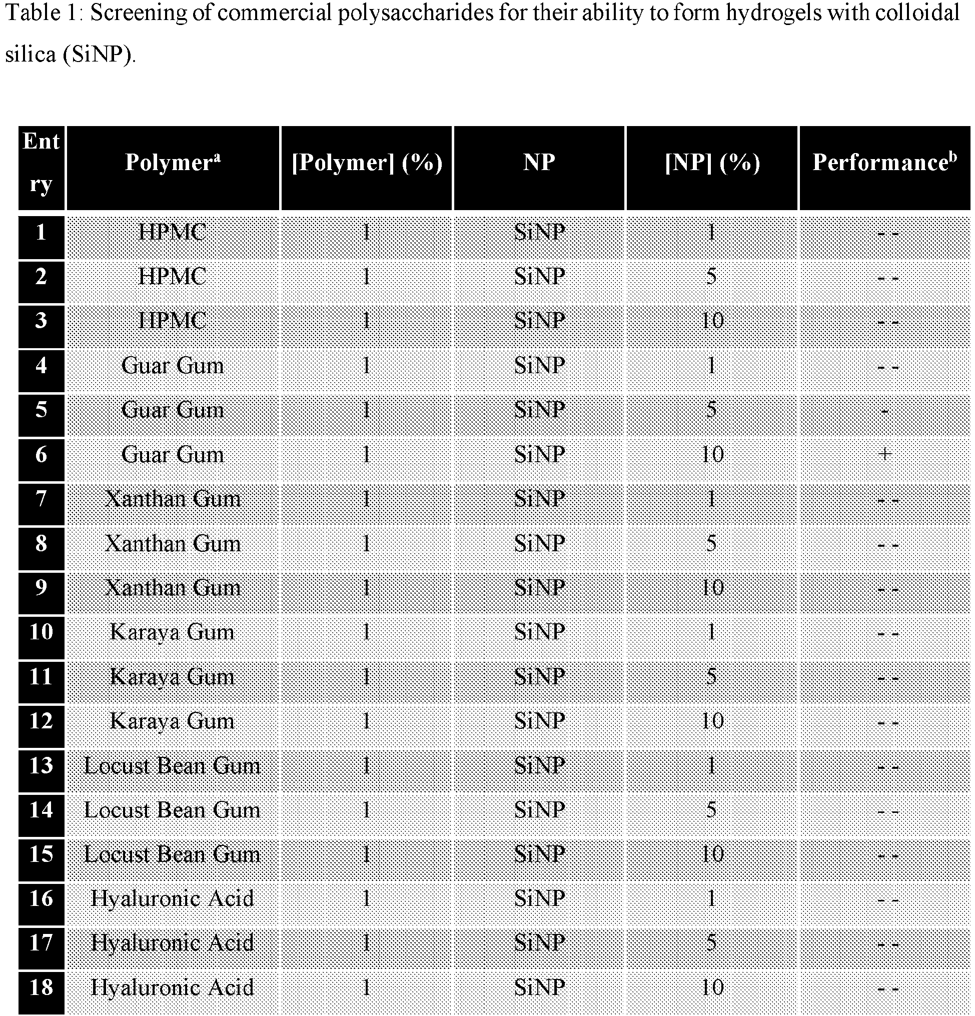

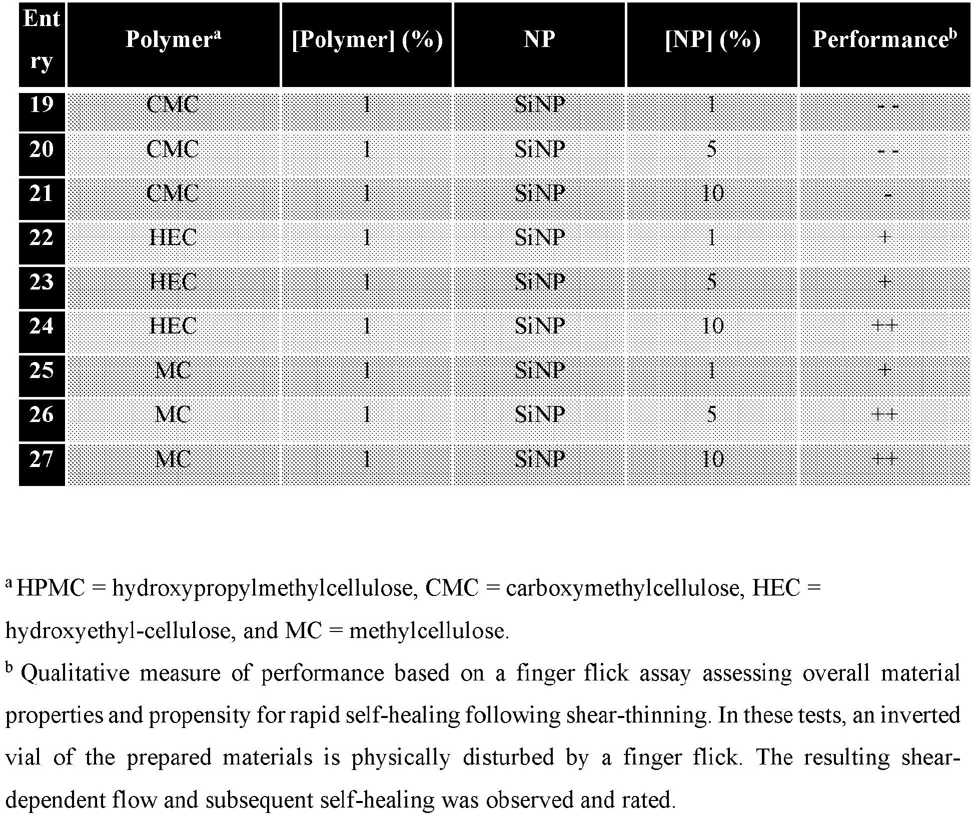

Subsequently a series of trimeric hydrogels with various combinations of HEC, MC and colloidal silica, as listed in Table 2, was prepared to optimize the formulation for initial mechanical properties, self-healing behavior and stability over time. In these experiments the total concentration of polysaccharides was maintained at 1 wt% while the colloidal silica particle concentration ranged from 1-5 wt%.

While all tested formulations demonstrated good-to-excellent mechanical properties and self-healing tendencies initially, as shown in Figure 2a, many of them suffered from syneresis over time, as shown in Table 2. Yet, at HEC:MC ratios of 80:20, strong, yet elastic hydrogels were formed with colloidal silica particles, and mechanical properties were maintained for at least one week, as indicated by a shear storage modulus of *G*' ~ 500 Pa (*ω =* 10 rad/s, *γ* = 2%) as a measure of hydrogel strength, and a high storage-to-loss oscillatory shear modulus ratio as a measure of hydrogel elasticity (with a loss oscillatory shear modulus G"; tanδ (tanδ = *G*"/*G*')*.*

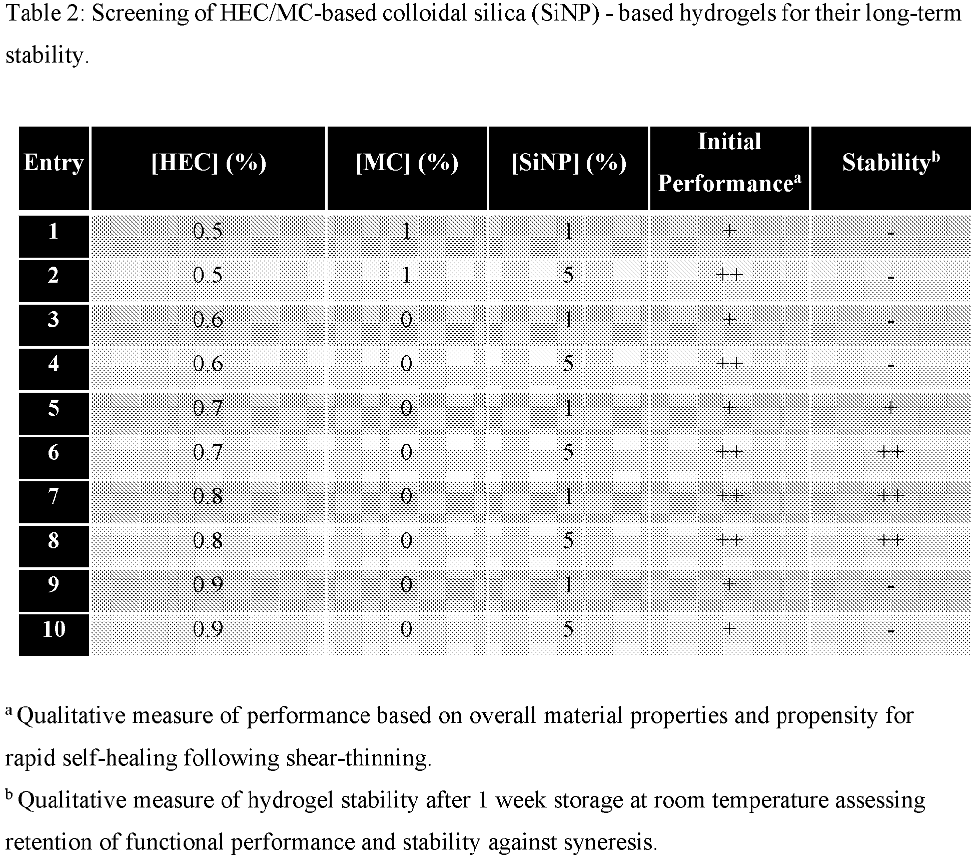

Interestingly, if colloidal silica particles of a different surface chemistry were used (Ludox TMA), the resulting hydrogels exhibited dramatically different mechanical properties, as shown in Figure 3. TMA-based materials, despite having only a slightly reduced G' value, exhibited a much higher tanδ value, and consequently, a much higher degree of fluidity. These data indicated that selective adsorption of the modified cellulose chains (in HEC and MC) to colloidal silica particles (CSP) enabled cross-linking and gel formation, supporting a physical picture of gel assembly.

### CONTRIBUTION OF POLYMER MOLECULAR WEIGHT

Using a formulation of HEC (0.8%) : MC (0.2%) : CSP (5%), the contribution of polymer molecular weight to the overall mechanical performance, including long-term stability, was investigated with cellulose silica-based hydrogels comprising combinations of high and low molecular weight HEC and MC polymers, as shown in Table 3.

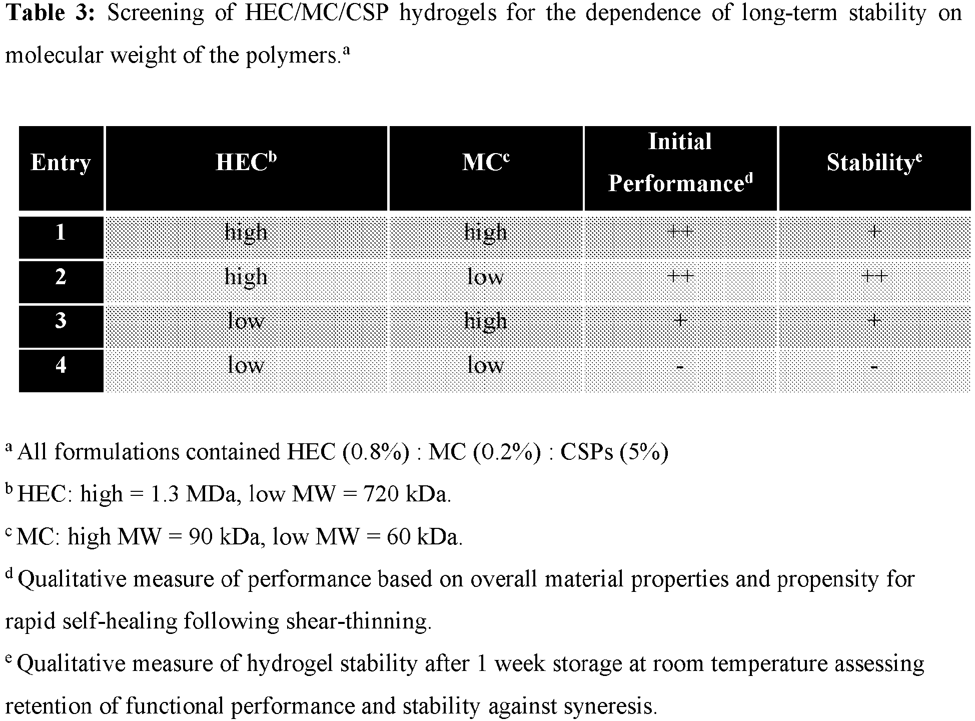

From these studies, it became apparent that HEC molecular weight was the primary determinant of hydrogel strength, whereby a decrease in molecular weight from *M*ᵥ ~ 1300 to *M*ᵥ ~ 720 kDa resulted in a dramatic decrease in material properties. In contrast, lowering the MC molecular weight from *M*ᵥ ~ 90 to *M*ᵥ ~ 60 kDa exhibited no significant decrease in the initial material properties, yet improved gel stability over time. While only two different molecular weights were examined for each polymeric component, these results indicate that polymer molecular weight can be exploited to tune gel properties.

Following the initial component screen, the impact of component loading and stoichiometric polymer-to-colloidal-silica particle ratio on the resulting hydrogel behavior was determined, as shown in Figure 2b. A stoichiometric polymer-to-colloidal-silica particle ratio of 1:5 maximized hydrogel strength and elasticity as indicated by a high value of shear storage modulus (G') and a low storage-to-loss oscillatory shear modulus ratio (tanδ), while deviation from the stoichiometric polymer-to-colloidal-silica particle ratio (either with more polymer or more CSPs) led to lower G' values and more fluid-like materials. Interestingly, increasing the overall loading of these components while maintaining the stoichiometric polymer-to-colloidal-silica particle ratio of 1:5 increased G' while leaving tanδ unchanged, indicating a maintenance of the overall degree of elasticity.

### SCALABILITY OF CELLULOSE SILICA-BASED TRIMER HYDROGELS

Based on these formulation results, the reproducibility and scalability of trimeric HEC:MC: colloidal silica particle (CSP) hydrogels were investigated. Hydrogels comprising HEC (1.2 wt%) : MC (0.3 wt%) : CSP (7.5 wt%) were prepared four times in parallel and the mechanical properties demonstrated excellent reproducibility, as shown in Figure 4. Subsequently their production was scalable in a linear fashion from 0.5 mL to over 15 L, as shown in Figure 2c, demonstrating the self-assembly approach as an extremely powerful and industrially scalable tool for trimeric HEC:MC:CSP hydrogels formation, as the manufacturing of these hydrogels can easily be scaled in a linear fashion over 4.5 orders of magnitude without resulting in an alteration of their mechanical properties.

Trimeric HEC:MC:CSP hydrogels are easily scalable because the gel formation and mechanical properties are solely dependent on the selective adsorption between cellulose derivatives and colloidal silica particles, which, on the molecular size-scale, is indifferent to total production volume.

As shown in Figure 2d, strain-dependent oscillatory rheology of trimeric HEC (1.2 wt%) : MC (0.3 wt%) : CSP (7.5 wt%) hydrogels displayed an extremely broad linear viscoelastic regime, and network failure occurring only at high strains (>20%), indicating a wide processing range and shear-thinning behavior. The frequency dependence of the storage and loss oscillatory shear moduli in the linear viscoelastic regime confirmed hydrogel-like behavior, as G' was dominant across the whole range of frequencies observed (0.1 - 100 rad/s, as shown in Figure 2e). Moreover, the observed shear-thinning behavior was corroborated with steady shear rheometrical measurements, as shown in Figure 2f, which indicated that the materials exhibit a power law decrease in viscosity with increasing shear rate.

### LIBRARY OF CELLULOSE SILICA-BASED TRIMER HYDROGELS WITH DIFFERENT PAIRS OF HYDROXYETHYLCELLULOS AND METHYLCELLULOSE

A library of cellulose silica-based trimer hydrogels was created with various combinations of HEC and MC as well as with HEC of various molecular weight to engineer a range of materials with highly disparate mechanical properties and tunable responsiveness to environmental conditions.

In these experiments the total concentration of polymer was maintained at 1 wt%, CSP concentration was kept at 5 wt%, and the ratio of HEC to MC was altered. According to frequency-dependent oscillatory rheological measurements performed in the linear viscoelastic regime, the shear storage modulus (G') was much greater than the loss modulus (G") throughout the entire observed frequency range, indicating hydrogel formation. As explained above, G' is reported as a measure of hydrogel strength, and tanδ (tanδ = G"/G') as a measure of hydrogel elasticity. As shown in Figures 3a-c, alteration of the formulation allows for tuning of G' from 10 to roughly 100 Pa with ω = 10 rad/s and γ = 2% simply by changing the ratio of HEC to MC while keeping the overall ratio of polymer to CSP ratio constant (polymer 1% : CSP 5%), or by changing the molecular weight of the HEC while keeping the formulation constant. The results in Figure 3b, furthermore, indicate that, while G' is modulated through alteration of the ratio of HEC to MC, the tanδ remains unchanged.

Strain dependent oscillatory rheology (Figure 3d-e) of these materials displayed an extremely broad linear viscoelastic regime and network failure only at high strains (>10%), indicating a wide processing range and shear-thinning behavior. Moreover, the observed shear-thinning behavior was corroborated with steady shear rheometrical measurements (Figure 4a-c), which indicated that the materials exhibit a power-law decrease in viscosity with increasing shear rate. The shear-thinning index reported in Figure 4 refers to the power law exponent from fitting of the viscosity versus shear rate obtained in a steady-shear measurement. Meanwhile the materials also exhibit rapid self-healing properties from the step-shear measurement (Figure 5) where the cellulose silica-based hydrogels go through two cycles of breaking and healing. Under a high shear rate, the materials break and show low viscosity, but under a subsequent low shear rate the materials completely restore to the original viscosity in less than 5 seconds, demonstrating rapid self-healing. When the hydrogel is sheared at a high rate, chains are placed under tension and desorb from the particles to relax stress, allowing the gel to flow. Upon relaxation of the applied shear, the hydrogels can rapidly re-form as polymers re-adsorb to particles in a new configuration.

Flow rheology was also characterized with yield-stress measurements. These materials exhibit shear-thinning behavior (Figure 4d-f, Figure 6). Importantly, both the magnitude of the viscosity and the degree of shear-thinning can be broadly tuned through alteration of the formulation.

The responsiveness of these materials was then tested in a number of ways. A temperature ramp of these gels in flow (γ = 0.5 s⁻¹; Figure 7) as well as steady-shear rheology of these gels at elevated temperature (Figure 8a) indicate that the mechanical response is completely unchanged by temperature. This insensitivity of the rheological properties to temperature is highly distinguishing from other reported non-covalently cross-linked systems that exhibit tremendous loss of mechanical stability with increasing temperature. As cross-linking in these other systems is strongly enthalpically driven, whereby association constants decrease significantly with increasing temperature, yielding a decrease in the density of effective crosslinks and thus a decrease in the mechanical properties. It is conceivable that the polymer-nanoparticle interactions exploited in this system are entropically driven, enabling complete stability to temperature over typical ranges.

These materials are completely stabile to increased ionic strength (2% KCl; Figure 8b), while they exhibit some pH responsiveness (Figure 8e and Figure 9). When the pH is increased above 9, the gels start to lose some of their integrity as the surface silanol groups on the CSPs are deprotonated above this pH.

The hydrogels formed by colloidal silica nanoparticles bearing different surface chemistry (Ludox TMA) with pairs of cellulose derivatives were qualitatively screened and found to exhibit dramatically different mechanical properties. In detail, the mechanical properties of cellulose silica-based hydrogels with 3 different kinds of colloidal silica nanoparticles (Ludox TMA, Ludox TM-50 and Ludox AS-40) were investigated (Figure 8d and Figure 10). Ludox TM-50 and Ludox AS-40 silica nanoparticles comprise anionic silonol groups on their surface in basic solution that are stabilized by sodium ions or ammonium ions, respectively. In contrast, Ludox TMA silica nanoparticles comprise cationic alumina on their surface that is stabilized with chloride ions.

The rheological characterization shows that cellulose silica-based hydrogels formed by AS-40 and TM-50 have identical mechanical properties in all the measurements shown in both flow and oscillatory rheology. However, TMA-based materials hardly formed gels and despite having only a slightly reduced G' value, exhibited a much higher degree of fluidity. Similarly, the hydrogels with TMA-based materials had a much lower yield stress than the hydrogels formed by AS-40 and TM-50. These data corroborate that the chains of the polymers (cellulose derivatives) absorb to select colloidal silica surfaces, which leads to the trimeric hydrogel formation; the counter ions, however, do little or nothing to impact this process.

### EXAMPLE 2: USE OF CELLULOSE SILICA-BASED HYDROGEL AS CARRIERS FOR PIPELINE PIGGING IN FOOD AND BEVERAGE MANUFACTURING

Industrial settings present many unique and complicated engineering challenges. Inefficiencies arise in manufacturing as large volumes of material need to be pumped from one location to another, and vast lengths of varying diameter pipe must be cleaned frequently (Tiratsoo, 1992). Applications as diverse as hydraulic fracturing and cosmetics rely on processable fluids with complex viscoelastic properties. Moreover, many products and coatings are applied through spraying, which often requires uniform application and tunable retention of solvent and/or product.

Pipeline pigging refers to the practice of using devices to perform various maintenance operations (*e.g*., cleaning or inspecting) on a pipeline without stopping the flow of the product in the pipeline (the name "pigging" arises from the squealing and snorting sound traditional mechanical "pigs" produce while traveling through a pipe) (Tiratsoo, 1992; Cordell & Vanzant, 2003). These processes have been used for many years to clean large diameter pipelines in the oil industry; however, the use of smaller diameter pigging systems has seen recent growth in industries handling products as diverse as paints, chemicals, and cosmetics to avoid cross-contamination and to increase product yields and reduce waste. Yet, for all of its benefits, the process is limited by the availability of materials amenable to both the product and pipeline systems at hand. For example, a pipeline cannot be "pigged" traditionally if it contains butterfly valves or reduced-bore ball valves, or comprises various sizes of piping (common in many older factories) (Cordell & Vanzant, 2003). Moreover, many standard pigging systems cannot be used with food products. Currently, most operators of batch processes in food production ensure maximum product recovery from a line by flushing the line with water (in some cases, this can sometimes be done with the next product). During these processes it is often necessary to downgrade or dump the contaminated or diluted portion of the product. Further, as many food products lead to pipeline fouling, cleaning agents (*e.g.,* hot caustic soda solutions) are often used, which must be subjected to effluent treatment or costly waste disposal (Gesan-Guiziou et al., 2002; Merin et al., 2002; Bremer et al., 2006). Many, if not all, of these problems could be eliminated with next-generation pigging systems, which would serve to both increase production and reduce the environmental impact of diverse batch operations.

One promising approach reported recently is based on the use of a slurry of ice for pigging, which could be easily applied in the food and beverage industries (Quarini, 2002; Quarini & Shire, 2007; Quarini et al., 2010). As these ice slurries are pumpable, they can form a soft plug capable of adapting its shape to fill complex pipe architectures, enhancing produce recovery and pipeline cleaning. However, ice production, transportation and storage is expensive, energy intensive, and poorly scalable. Moreover, as ice melts rapidly when present in small particles, the distance over which an ice slurry may function as an effective "pig" is severely limited (Evans et al., 2008; Hales et al., 2014). This is particularly problematic in industries where most product movement occurs during the summer months (*e.g*., wine production).

Scalable and moldable cellulose silica-based trimeric hydrogels that were prepared from environmentally safe, cost-effective and renewable polysaccharide starting materials, as described in Example 1, were applied to address the particular challenge of pipeline maintenance in large-scale wine production. Large-scale wine production is highly dependent on product transfer through diverse pipeline systems during the many stages of production, including crushing of the fruit, fermentation, aging, and bottling, as shown in Figure 5a. According to the Wine Institute, wine production in the U.S. is approaching 1 Billion gallons/year and anecdotal evidence from within the industry indicates that up to 2% of product may be lost during production. Indeed, in large wineries, over 20 cases of wine may be lost per transfer. Besides an acceptable safety profile both for use in food products and for the environment, a viable pigging system must be tasteless, scentless, and colorless to avoid any perturbation of the product.

### Cellulose silica-based trimer hydrogels are tasteless, scentless and odorless

As all components of the scalable and moldable cellulose silica-based trimer hydrogels are highly pure, chemically simple, and widely utilized in food production, a blind taste test was performed with three blinded professional wine makers. For these experiments, one of three identical samples of wine was mixed with a cellulose silica-based trimer hydrogel to a concentration of approximately 1 wt% hydrogel. Three blinded professional wine makers then tested all samples of wine in an attempt to identify the sample that contained the cellulose silica-based trimer hydrogel, but none of them detected a statistically significant difference with respect to taste, scent or odor confirming that cellulose silica-based trimer hydrogels are tasteless, scentless and odorless. Consequently, selected cellulose silica-based trimer hydrogel formulations were investigated for pipeline pigging both in the recovery of product and in pipeline cleaning.

Initially, a trimeric hydrogel formulation with HEC (1.2 wt%) : MC (0.3 wt%) : CSP (7.5 wt%) was tested for pipeline cleaning applications. This formulation was chosen on account of its material properties (maximizing G' and minimizing tanδ) and low content of solids (comprising 91% water). Laboratory-scale experiments were designed to emulate current approaches in batch processes in industrial-scale food and beverage manufacturing. A test pipe (*d =* 1/2", *l* = 24") was allowed to foul with Purple 8000 crushed-grape concentrate (10% v/v) for 1 h. Then, either KOH (1M; industry standard) or cellulose silica-based hydrogel was pumped through the pipe (at a rate of 15 mL/min) to remove the grape residue. As seen in Figure 5b, cellulose silica-based hydrogel provides greatly enhanced scouring of the piping over the industry standard, which often requires soaking for several hours before flushing through the pipe to effectively dislodge grape residue. Aqueous solutions of Purple 8000 grape concentrate (10% v/v) were then propelled through a test pipe (*d =* 1/2", *l* = 24") with one pipe volume (~77mL) of either water (consistent with industry standard practice) or cellulose silica-based hydrogel. By monitoring the absorbance of the Purple 8000 (λₘₐₓ = 535 nm) in the eluent over time, it was possible to determine the recovery of Purple 8000 from the pipeline (using a cut-off at dilution to 80%, as shown in Figures 5c-e).

Cellulose silica-based hydrogel formulations created a defined interface with the model product, allowing for greatly enhanced product recovery over the industry standard and demonstrating that these hydrogel formulations can be used with industry standard pumps (e.g., progressive cavity pumps, peristaltic pumps, or diaphragm pumps). These results establish that cellulose silica-based hydrogel formulations can serve as scalable, food-safe, environmentally benign and water-wise options for pipeline pigging in industrial scale food and beverage manufacturing.

### EXAMPLE 3: USE OF CELLULOSE SILICA-BASED TRIMERIC HYDROGELS AS CARRIERS OF FIRE RETARDANTS FOR ENHANCED FIRE RETARDANT PERFORMANCE AND RAINFASTNESS TO PREVENT WILDLAND FIRES BY PRETREATING HIGH-RISK AREAS AND TO DIRECTLY FIGHT WILDLAND FIRES

According to the National Interagency Fire Center (NIFC), wildland fires in the U.S. destroy almost 10 million acres/year and the cost of fighting wildland fires in the U.S. is estimated to be roughly $2B annually. The NIFC is a federal organization coordinating the efforts of multiple federal agencies, including the Bureau of Land Management, Bureau of Indian Affairs, US Fire Service, US National Park Service, US Fish & Wildlife Service, and the US National Association of State Foresters.

Fire retardants, typically comprising ammonium polyphosphates in aqueous formulation, constitute a primary tactical resource in fighting wildland fires, with over 30 million gallons pumped annually in the US alone (Gimenez et al., 2004; Yount, 2015). Yet, their tactical utility is severely limited by their current formulations which must often be dropped from low altitudes on account of drift or rapid evaporation while falling, or are incapable of retaining these retardants at the target site.

Another class of highly effective retardants are aqueous film forming foams, which comprise perfluorinated surfactants, the primary fire extinguishing chemical driving their performance (Kissa, 1994). However, wastewater from the use of these foams and its treatment have been the focus of rigorous investigative studies as fluorinated surfactants have been found to contaminate groundwater around the site of application and have emerged as primary environmental contaminants due to their environmental persistance, potential for bioaccumulation, and toxicity (Kissa, 1994; Moody & Field, 2000; Moody et al., 2002; 2003; Hu et al., 2016). The use of these foams is therefore highly restricted.

Furthermore, "water-enhancing gels," which are aqueous dispersions of super-absorbent polymers similar to the materials used in disposable diapers, have demonstrated the capacity to extend water's effectiveness in preventing fires from starting and/or spreading (Schroeder, 2005; 2006a; 2006b). These water-enhancing gel materials, however, are non-degradable and petroleum-derived, posing significant environmental concerns and limiting their applicability.

### Cellulose silica-based trimeric hydrogels enhance fire retardant performance and fire retardant's rainfastness

Scalable and moldable cellulose silica-based trimeric hydrogels that can be prepared from environmentally safe, cost-effective and renewable polysaccharide starting materials present a unique solution to these important industrial and environmental challenges and limitations because they are capable of imparting enhanced drop capabilities such as reduced drift, greater canopy penetration, and the potential to be dropped from greater altitudes, along with the ability to retain ammonium polyphosphates at the target site, which makes cellulose silica-based hydrogel carriers more effective and safer tactical approaches to fighting wildland fires than the presently used methods. Furthermore, enhanced retention of fire retardants with cellulose silica-based hydrogel carriers would enable application at sites of particularly high fire danger (*e.g*., roadsides or targets for lightning strikes), potentially preventing a vast number of wildland fires from starting in the first place.

Select cellulose silica-based hydrogel formulations (HEC (0.32 wt%) : MC (0.08 wt%) : CSP (2 wt%)) were formulated with Phos-Chek LC95A, an ammonium polyphosphate retardant that is typically dispersed in water. Phos-Chek LC95 is the most commonly used fire retardant in the US with approximately 15 million gallons pumped annually (Yount, 2015).

For sprayable formulations, cellulose silica-based trimer hydrogel preparations with the same component stoichiometry as above were prepared, but at a lower concentration so that the hydrogel preparations could be sprayed like standard aqueous formulations of the Phos-Chek LC95 fire retardant, but that the hydrogel preparations yet would have the potential to provide better canopy penetration and substantially enhanced persistence at the site of application, properties that are critical for fire prevention in high-risk areas.

Although current fire retardant formulations include small amounts of thickening agents (e.g., Palygorskite clay), their efficacy is completely neutralized by subsequent drops of water (limiting tactical approaches to directly fighting fires) or even by minimal rainfall (precluding preemptive use).

In the investigation of cellulose silica-based trimer hydrogel preparations as carriers for fire retardants, pinewood flour, which is a model high-surface-area to volume fuel, was treated with the hydrogel preparations in various ways, and thermal gravimetric analysis (TGA) was performed to characterize mass loss upon burning, as shown in Figure 13b. TGA was performed on account of its prevalence in studies regarding material ignition and decomposition processes as well as quantification of flame retardant performance (Drevelle et al., 2005; Gu et al., 2007).

Both the mass remaining after the initial burn and the persistence of mass over time for untreated wood was determined as well as for wood treated with hydrogel alone, for wood treated with aqueous retardant, and for wood treated with retardant-containing hydrogel, as shown in Figure 13c. When comparing untreated wood and wood treated with standard aqueous retardant formulations, it is apparent that the fire retardant decreases both the initial mass loss and inhibits subsequent mass loss. The addition of hydrogel in either case did not significantly alter the performance of the retardant.

However, when wood samples treated with standard aqueous retardant formulation and retardant-containing hydrogel formulations were subjected to simulated rainfall, their subsequent performances differed dramatically, as shown in Figure 13d. To simplify identification of the performance of the treatments (standard aqueous retardant formulation versus retardant-containing hydrogel formulations) following simulated rainfall, the TGA data were normalized to reflect the response of wood alone (yielding a performance rating of 0%) and wood freshly treated with standard aqueous retardant formulation (yielding a performance rating of 100%).

When fire retardant-containing cellulose silica-based hydrogel was applied to "one-hour" fuel, as illustrated in Figure 14a, the fire retardant-gel combination enhanced adherence of the retardant treatment to the fuel which resulted in a 95% surface coverage, while the surface coverage of standard retardant treatment was only 80%. When the treated "one-hour" fuel was burned in a test chamber, the enhanced surface coverage and enhanced adherence of the retardant when applied as fire retardant-gel led to longer born duration (Figure 14b/c), i.e. slower heat release, and lower maximum temperatures (Figure 14c).

To further test the enhanced rainfastness of fire retardant-hydrogel preparations, a rain event was simulated that corresponded to approximately 0.25" of rain-fall on fuels treated with retardant/hydrogel and standard retardant formulations, respectively, using a backpack sprayer. Following the rain event, the standard retardant formulations were washed away, as evidenced by the red tinted of the run-off water (Figure 15a), while the gel-based application resulted in complete retention of the retardant on the fuel, as subsequent burning in a test chamber showed. Burning these samples (1 kg) in a test chamber indicated enhanced persistence of the fire retardant on the fuel throughout the rain event by longer burn duration (Figure 15b/c) and lower maximum temperatures (Figure 15c).

These results clearly show that hydrogel formulations retain the fire retardants on the fuel throughout the simulated rainfall, while standard formulations are washed away, losing their performance, as shown by a loss of functional performance following a subsequent burn (Figure 15c). In contrast, the fire retardant-hydrogel-based application resulted in complete retention of the retardant on the fuels, which led to complete maintenance of retardant performance. While these gel materials can be formulated to persist for several months in the environment, even through small rain events, their physical nature will allow them to completely biodegrade in the environment after the fire season. Therefore, prophylactic treatment of areas at high-risk for fire starts (e.g., roadsides) with fire-retardant-hydrogel formulations will be instrumental in preventing fires from starting in the first place.

### EXAMPLE 4: VARIOUS FORMULATIONS OF CELLULOSE SILICA-BASED TRIMERIC HYDROGELS AS CARRIERS OF FIRE RETARDANTS FOR ENHANCED FIRE RETARDANT PERFORMANCE

As described above in Example 3, hydrogel formulations that function as carriers for fire-retardants have the potential to dramatically enhance retardant persistence at the site of application while allowing for facile application through standard spray or drop approaches. Five different hydrogel formulations were created, and their viscoelasticity and shear-dependent viscosity was characterized based on frequency-dependent as well as flow rheometry, as shown in FIGS. 16a,b , FIG. 17 and FIG. 18. By changing the HEC/MC ratio or colloidal silica nanoparticle (CSNP) amount, the mechanical properties were broadly tunable to exhibit a range of G', tan(d), shear-dependent viscosity, and flow profiles, see FIGS. 17 and 18.

### Materials

Hydrogels were prepared with 0.1 or 0.2 wt% HEC/MC (0.85/0.15) and 0.5, 1, or 2 wt% colloidal silica nanoparticles (CSNPs). Ammonium polyphosphate was loaded into the gels at 13.5 wt%, consistent with PC (commercial standard) formulations.

All rheometry experiments were performed on a torque controlled Discover HR2 Rheometer (TA Instruments). Dynamic measurements on hydrogels were performed using a 60 mm cone plate (Peltier plate steel; 2.007°) from 0.1 rad/s to 100 rad/s. The applied torque required to stay in the linear viscoelastic region was obtained from amplitude sweeps conducted for each formulation. Steady shear experiments were performed using the same geometry and were done from 0.1 to 100 s⁻¹.

The chemical oxygen demand (COD) and biochemical oxygen demand (BOD) of the HEC/MC and HEC/MC/CSNP mixtures were determined according to standard methods (Rice et al., 2017). The seed was domestic wastewater that was aerated for 24 hours prior to the test. The positive control used in the BOD test was a mixture of glucose and glutamic acid (BOD Standard Solution, Hach, Loveland, CO). For anaerobic biodegradability, a biochemical methane potential (BMP) assay based on reported protocols was used (Labutat et al., 2011). The concentration of polymer mixtures used was 1.8 mg COD/L. The inoculum used was effluent from a wastewater treatment plant anaerobic digester (Oceanside Water Pollution Control Plant, San Francisco, CA). The nutrient/trace metal media was adopted from reported protocol. The total volume of the polymer mixture, inoculum, and nutrient/trace metal solution was 100 mL and was sealed in 160 mL serum bottle with butyl-rubber stoppers. The headspace was flushed with nitrogen gas to establish anaerobic conditions. Triplicate bottles were incubated at 37°C for thirty days and gas chromatography was used to determine methane concentrations of the produced gas. Gas production from blanks consisting of only inoculum was subtracted from each sample.

For laboratory water drop tests, 1 mL of PC and 1mL of each hydrogel formulation, respectively, loaded with ammonium polyphosphate were pipetted onto a glass slide and allowed to dry overnight. These glass slides were then placed at a ~50° incline and water was dripped onto the dried sample from a nozzle ~1.3 cm above the slide. The syringe pump was set at 5 mL/min, and a total of 20 mL of water was used.

### Formulation 1 as best performer

**Formulation 1** (0.17:0.03:1 HEC:MC:CSNP wt%; **"GEL1" or "1")** ultimately exhibited the best overall performance in laboratory-scale burn experiments shown below and exhibited a slightly higher modulus than the commercial standard, **"PC"**. Markedly, while PC showed a crossover frequency at ~0.4 rad/s, formulation 1 had no crossover frequency throughout the entire range tested (FIG. 16A). Furthermore, the higher moduli values at lower frequencies suggested that formulation 1 had a higher solid-like behavior at longer time scales when compared to PC. This difference became manifest in simulated rain experiments on samples dried on glass slides (FIG. 16F). After 20 mL of water was dropped onto the glass slides placed at an incline, formulation 1 exhibited no adhesive or cohesive failure of the material, while the PC sample completely washed off along the flow pathway. Despite this solid-like behavior of formulation 1, steady shear experiments illustrated that formulation 1 exhibited viscosities that were similar to those of the commercial standard (PC) and had comparable shear-thinning behavior (FIG. 16B and FIG. 18). The similarity in viscosity and shear-thinning behavior indicates that formulation 1 can be sprayed and thus be deployed using conventional methods.

### Biological Impact Of Hydrogel Formulations

Cell toxicity and biodegradability studies were performed to investigate the biological impact of these hydrogel formulations. Measurements of relative cell apoptosis revealed that hydrogels formed with cell media displayed no significant changes in cell apoptosis compared to the control for a fully constituted hydrogel formulation and across all dilutions measured (see FIG. 16C). In order to determine the anaerobic and aerobic biodegradability of the hydrogel formulations, the biochemical oxygen demand (BOD) and the chemical oxygen demand (COD) of the hydrogel formulations with CSNPs (HEC/MC-CSNP) and without CSNPs (HEC/MC) were assessed, according to standard methods (Rice et al., 2017). The BOD tests showed that both HEC/MC-CSNP and HEC/MC as well as a blank consisting solely of the seed and nutrients reached a dissolved oxygen concentration of ~4 mg/L at the end of a 24-day period, with an increase in dissolved oxygen consumption at day 12 due to ammonium oxidation (FIG. 16D). Only the positive control, consisting of glucose and glutamic acid, showed significant oxygen depletion relative to the blank. These results suggested that the hydrogel formulations did not display any toxicity or inhibition of microbial activity. Importantly, the absence of an additional oxygen demand for the hydrogel formulations suggests that these materials are unlikely to contribute to organic pollution in the environment. Similarly, the biochemical methane production (BMP) experiments revealed that the hydrogel formulations were slightly resistant to microbial degradation and did not immediately degrade when exposed to microbes (FIG. 16E). There was no significant difference in methane production between the HEC/MC-CSNP and HEC/MC formulations, with both reaching ~14 L CH₄/kg COD at the end of the 30-day period. The detection of methane in each formulation indicated that the material components did not inhibit methanogenic activity and did not rapidly degrade. The slow rate of degradation prevent the hydrogels from deteriorating during the fire season which can persist for upwards of 7-8 months, while also preventing rapid depletion of oxygen from the surrounding environment once washed away.

### Fire Retardant Retention

Laboratory-scale burn experiments were performed to determine the ability for retardant retention in the environment after small rain events. Although fire retardant burn experiments are often performed in cone calorimeters, these methods are unable to capture the dynamics of a spreading fire front and are limited to small amounts of fuel (Finney et al., 2015). Therefore, model burn chambers were established for each fuel type to account for at-scale burn dynamics and masses. Specifically, two fuel types notorious for fire starts were chosen: (i) chamise (greasewood) chipped to be a "1-hour fuel" and (ii) hay, which is a "light, flashy fuel."

For chamise chip burns, the burn chambers (*l* = *w* = 15 cm, *h* = 43 cm) were fitted with an ignitor and thermocouple at the base and two thermocouples at the fuel fill line. Untreated chamise chips were placed at the bottom of the chamber, with treated chamise filling the rest of the chamber. Quantification of the area under the temperature versus time curves for chamise chip burns demonstrated that rain substantially reduced the ability of the commercial standard (PC) to suppress the spread of fire (FIG. 20A and FIG. 21). After 0.5" of rain, the PC treatment performed no better than the untreated control. Yet, in the case of formulation 1 ("1"), the efficacy remained consistent even up to 0.5" of rain, which is typically the high point of rain that occurs during a fire season (FIG. 20B). For direct comparisons against PC, the performance of the hydrogel formulations was normalized such that 100% corresponded to PC treatments with no rain. As rain was applied to PC-treated chamise, the performance of PC dropped to <50% after 0.25" rain and to 0% after 0.5" rain, while the formulation-1 treated chamise retained performance superior to PC (>100% performance) for all rain groups (FIG. 20C).

Hay burns were executed in burn chambers (height ~12.5 cm, diameter ~11 cm) that were fitted with an ignitor at the base and three thermocouples-two at the top of the fuel fill line and one at the ignitor. Similar to the chamise chip burns, hay burn experiments illustrated that rain dramatically reduced the retardant efficacy of the commercial standard PC, while it only minimally affected the retardant efficacy of formulation 1, as shown with formulation-1 treated hay (FIGS. 20D-F and FIG. 22). Notably, formulation-1 treated hay without rain experienced essentially no ignition among the samples tested, resulting in no appreciable heat released or mass consumed (FIG. 20E and FIG. 22C).

### Pairing Of Superior Retention With Homogeneous Coatability

To achieve an optimally fire-retarding effect it was important that the hydrogel formulations not only exhibited superior fire retardant retention characteristics, but also possessed the ability to homogeneously coat the fuel to maximize performance. While the formulations 2-5 demonstrated similar retention enhancing properties, the burn data as well as the rheological characterization of the formulations indicated that formulation 1 was the most promising. Formulation 1 was therefore used in all subsequent experiments (FIGS. 17, 18, and 20).

### Pilot-Scale Burn Experiments

Chamise piles (100 kg of material) treated with CL2 of gel treatment **1** and exposed to 0.5 inches of rain were burned. All burns were started by ignition of an untreated starter bundle of chamise (United States Forest Service). Untreated control piles rapidly ignited and grew to a steady-state burn temperature after ~180 seconds, while **1** treated piles delayed ignition and flame growth until ~400 seconds to reach the same burn temperatures (Fig. 20). The slower rate of ignition and transition of the flame from the untreated starter bundle to the treated pile is due to the intumescent effects of the ammonium polyphosphate loaded in **1.** Nonetheless, for these pilot-scale burns, the impact of the fire retardants was largely observed during early phase of the burns and once the fires matured (>420 seconds), the fire overcomes any intumescent effects and burn normally, resulting in similar flame sizes and average temperatures across all treatments.

### Formulation 1 Coverage Level Required To Minimize Burning

The coverage level of formulation 1 that was needed to minimize burning in pilot-scale plots of standing and mowed dry grass was tested next, because standing and mowed dry grass represent the conditions of actual roadsides in many locations within the U.S.

In cases of direct application (no rain) of formulation 1 on mowed grass, the flame stopped immediately after ignition, as observed for all coverage levels, and burned <10% of the total plot area, while the untreated control burned ~90% of the area within 24 seconds (FIG. 23). After simulating 0.5" of rain on each coverage level, results indicated that CL3 or higher was necessary to completely preclude the spread of the flame and reduce the burned area to <10% (FIGS. 24A, B). These results demonstrate that CL1 was sufficient to completely stop spreading of the fire, while after 0.5" of rain, CL3 was sufficient. Analogous results were seen for standing grass pilot-scale plot, where direct application of CL1 was enough to completely stop the active flaming front (FIG. 25).

Overall, the burn experiments demonstrate the efficacy of the tested hydrogel formulations to prevent and slow wildfires. This materials-based strategy has the potential to be used prophylactically at high risk areas, potentially protecting the lives and infrastructure of local populations, while reducing environmental pollution. The combination of unique and tunable mechanical properties, inexpensive and renewable starting materials, scalable and inexpensive manufacturing, and enhanced retention of fire retardants makes these materials well positioned to impact the pressing challenges in wildfire prevention and suppression.

### EXAMPLE 5: USE OF CELLULOSE SILICA-BASED TRIMERIC HYDROGELS AS CARRIERS OF FERTILIZERS AND PESTICIDES

Cellulose silica-based trimeric hydrogel formulations are not only suited to serve as easily applicable and well-retaining, i.e. not quickly washed-away, carriers for the delivery of fire retardants, as described in Example 3, but also suited to serve as such carriers for the controlled delivery of fertilizers such as ammonium polyphosphates (which was described in Example 3 as common ingredient in fire retardants) and pesticides.

Since, as explained in Example 3, such hydrogel formulations are easily applicable as sprayable carriers, but not readily washed away by light rain, they can be applied onto crops and other materials with enhanced surface coverage and enhanced fertilizing and pesticidal performance, respectively. Since such hydrogel formulations are not readily washed away by light rain, their use as carriers for pesticides and fertilizers will result in less groundwater contamination through washed-away pesticides and fertilizers.

### REFERENCES

Appel EA, del Barrio J, Loh XJ, & Scherman OA (2012a) Supramolecular Polymeric Hydrogels. Chem. Soc. Rev. 41:6195--6214.
Appel EA, et al. (2015a) Self-assembled hydrogels utilizing polymer-nanoparticle interactions. Nature Commun. 6:6295*.*
Appel EA, et al. (2015b) Exploiting Electrostatic Interactions in Polymer-Nanoparticle Hydrogels. Acs Macro Lett 4(8):848-852.
Bergna HE & Roberts WO (2005) Colloidal Silica: Fundamentals and Applications (Taylor & Francis Group, New York).
Cordell J & Vanzant H (2003) The Pipeline Pigging Handbook (Clarion Technical Publishers and Scientific Surveys Ltd.) 3rd Ed.
Drevelle C, et al. (2005) Thermal and fire behaviour of ammonium polyphosphate/acrylic coated cotton/PESFR fabric. Polym Degrad Stabil 88(1):130-137.
Evans TS, Quarini GL, & Shire GSF (2008) Investigation into the transportation and melting of thick ice slurries in pipes. Int J Refrig 31(1): 145-151.
Finney MA, et al. (2015) Role of buoyant flame dynamics in wildfire spread. Proc Natl Acad Sci USA 112: 9833-9838.
Gesan-Guiziou G, Boyaval E, & Daufin G (2002) Nanofiltration for the recovery of caustic cleaning-in-place solutions: robustness towards large variations of composition. J Dairy Res 69(4):633-643.
Merin U, Gesan-Guiziou G, Boyaval E, & Daufin G (2002) Cleaning-in-place in the dairy industry: criteria for reuse of caustic (NaOH) solutions. Lait 82(3):357-366.
Bremer PJ, Fillery S, & McQuillan AJ (2006) Laboratory scale Clean-In-Place (CIP) studies on the effectiveness of different caustic and acid wash steps on the removal of dairy biofilms. Int J Food Microbiol 106(3):254-262.
Gimenez A, Pastor E, Zarate L, Planas E, & Arnaldos J (2004) Long-term forest fire retardants: a review of quality, effectiveness, application and environmental considerations. Int J Wildland Fire 13(1):1-15.
Gu JW, Zhang GC, Dong SL, Zhang QY, & Kong J (2007) Study on preparation and fire-retardant mechanism analysis of intumescent flame-retardant coatings. Surf Coat Tech 201(18):7835-7841.
Hales A, et al. (2014) Ice fraction measurement of ice slurries through electromagnetic attenuation. Int J Refrig 47:98-104.
Hu XC, et al. (2016) Detection of Poly- and Perfluoroalkyl Substances (PFASs) in U.S. Drinking Water Linked to Industrial Sites, Military Fire Training Areas, and Wastewater Treatment Plants. Environ. Sci. Technol. Lett. article.
Kapsabelis S & Prestidge CA (2000) Adsorption of ethyl(hydroxyethyl)cellulose onto silica particles: The role of surface chemistry and temperature. J Colloid Interf Sci 228(2):297-305.
Kissa E (1994) Fluorinated surfactants : synthesis, properties, applications (M. Dekker, New York) pp vii, 469 p.
Labutat RA, Angenent LT, & Scott NR (2011) Biochemical methane potential and biodegradability of complex organic substrates. Bioresour. Technol. 102(3):2255-2264.
Moody CA & Field JA (2000) Perfluorinated surfactants and the environmental implications of their use in fire-fighting foams. Environ Sci Technol 34(18):3864-3870.
Moody CA, Martin JW, Kwan WC, Muir DCG, & Mabury SC (2002) Monitoring perfluorinated surfactants in biota and surface water samples following an accidental release of fire-fighting foam into Etobicoke Creek. Environ Sci Technol 36(4):545-551.
Moody CA, Hebert GN, Strauss SH, & Field JA (2003) Occurrence and persistence of perfluorooctanesulfonate and other perfluorinated surfactants in groundwater at a fire-training area at Wurtsmith Air Force Base, Michigan, USA. J Environ Monitor 5(2):341-345*.*
Quarini J (2002) Ice-pigging to reduce and remove fouling and to achieve clean-in-place. Appl Therm Eng 22(7):747-753.
Quarini J & Shire S (2007) A review of fluid-driven pipeline pigs and their applications. P I Mech Eng E-J Pro 221(E1): 1-10.
Quarini G, et al. (2010) Investigation and development of an innovative pigging technique for the water-supply industry. P I Mech Eng E-JPro 224(E2):79-89.
Rice EW, et al. (2017) Standard Methodsfor the Examination of Water and Wastewater (American Public Health Association, American Water Works Association, and Water Environmental Federation, Washington DC, 2017).
Rodell CB, et al. (2015) Shear-Thinning Supramolecular Hydrogels with Secondary Autonomous Covalent Crosslinking to Modulate Viscoelastic Properties. Adv Funct Mater 25(4):636-644.
Rose S, et al. (2014) Nanoparticle Solutions as Adhesives for Gels and Biological Tissues. Nature 505:382--385.
Schroeder D (2005) Can Fire Suppressant Gels Protect Log Decks? A Case Study to Test the Concept. (Vancouver, Canada).
Schroeder D (2006a) Can Fire Suppressant Gels Protect Log Decks? Part III - Two case studies to test gel effectiveness against radiant and convective heat transfer. (Vancouver, Canada).
Schroeder D (2006b) Effectiveness of Forest Fuel Management: A Crown Fire Case Study in the Northwest Territories, Canada. (Vancouver, Canada).
Tamesue S, et al. (2013) Linear versus Dendritic Molecular Binders for Hydrogel Network Formation with Clay Nanosheets: Studies with ABA Triblock Copolyethers Carrying Guanidinium Ion Pendants. Journal of the American Chemical Society 135(41):15650-15655.
Tiratsoo JNH (1992) Pipeline pigging technology (Gulf Pub. Co., Houston) 2nd Ed, 460 p.
United States Forest Service (2006) "Coverage Levels" https:/ /www.fs.fed.us/rm/fire/pubs/pdfpubs/user _gd/ug-06. pdf.
Wang Q, et al. (2010) High-Water-Content Mouldable Hydrogels by Mixing Clay and a Dendritic Molecular Binder. Nature 463(7279):339--343.
Yount B (2015) Summary of National Retardant Use for 2015.

## Claims

1. A non-covalently crosslinked trimeric hydrogel
comprising
hydroxyethylcellulose (HEC),
methylcellulose (MC), and
colloidal silica nanoparticles,
wherein the HEC and MC are cross-linked by the colloidal silica nanoparticles to form the non-covalently cross-linked hydrogel, and wherein: the silica surface comprises at least 80% silanol groups and the silica surface is negatively charged in a pH dependent manner.

2. The hydrogel of claim 1, wherein:
the total proportion of HEC and MC is from 0.1 wt% to 0.2 wt% of the hydrogel,
the total proportion of colloidal silica nanoparticles is from 0.5 wt% to 2 wt% of the hydrogel, and
the weight ratio of HEC to MC is 85:15.

3. The hydrogel of claim 1, wherein said nanoparticles have a silica surface substantially consisting of silanol groups and said nanoparticles are negatively charged as a function of alkalinity.

4. The hydrogel of claim 1 being capable of linear scaling up while remaining capable of shear-thinning and self-healing and/or maintaining controllable mechanical properties between temperatures of 20 to 70 degree Celsius.

5. The hydrogel of claim 1, furthermore comprising at least one agent selected from a group of small molecules, organic molecules, inorganic molecules, cosmetic agents, pharmacologically active compounds, microorganisms, nucleic acid, peptides or proteins.

6. The hydrogel of claim 5 wherein said agent is a fire retardant agent, fertilizer or pesticide.

7. Use of the hydrogel of claim 5 as an aqueous viscosity modifier or for use in pipeline maintenance and product recovery.

8. Use of the hydrogel of claim 6 as carrier of fire retardants.

9. Use of the hydrogel of claim 6 as carrier of pesticides, for use as carrier of fertilizers, for use as a biomimetic scaffold in tissue engineering, or for use in drug and vaccine delivery.

10. A method of preparing the hydrogel of claim 1, said method comprising contacting a plurality of said colloidal silica nanoparticles and said HEC and MC in such a way that said HEC and MC can selectively adsorb to said colloidal silica nanoparticles via multivalent and non-covalent interaction and crosslinking.

11. A kit for forming the hydrogel of claim 1, the kit comprising a plurality of said colloidal silica nanoparticles and said HEC and MC.

12. A method of fire prevention, the method comprising contacting material prone to catching fire with the hydrogel of claim 6.

13. A method of pipeline pigging, the method comprising contacting pipelines in need of cleaning or purging with the hydrogel of claim 1 or 5.

14. A method of applying a chemical treatment in a controlled fashion, the method comprising contacting material in need of chemical treatment with the hydrogel of claim 5 or claim 9.

## Patentansprüche

1. Nicht-kovalent vernetztes trimeres Hydrogel
bestehend aus
Hydroxyethylcellulose (HEC),
Methylcellulose (MC) und
kolloidalen Siliziumdioxid-Nanopartikeln,
wobei die HEC und die MC durch die kolloidalen Siliziumdioxid-Nanopartikel vernetzt sind, um das nicht-kovalent vernetzte Hydrogel zu bilden, und wobei:
die Siliziumdioxid-Oberfläche mindestens 80 % Silanolgruppen umfasst und die Siliziumdioxid-Oberfläche pH-abhängig negativ geladen ist.

2. Hydrogel nach Anspruch 1, wobei:
der Gesamtanteil an HEC und MC 0,1 Gew.-% bis 0,2 Gew.-% des Hydrogels beträgt,
der Gesamtanteil an kolloidalen Siliziumdioxid-Nanopartikeln 0,5 Gew.-% bis 2 Gew.-% des Hydrogels beträgt, und
das Gewichtsverhältnis von HEC zu MC 85:15 beträgt.

3. Hydrogel nach Anspruch 1, wobei die Nanopartikel eine Siliziumdioxid-Oberfläche aufweisen, die im Wesentlichen aus Silanolgruppen besteht, und die Nanopartikel als Funktion der Alkalinität negativ geladen sind.

4. Hydrogel nach Anspruch 1, das linear skalierbar ist und dabei seine Scherverdünnungsfähigkeit sowie Selbstheilungsfähigkeit beibehält und/oder kontrollierbare mechanische Eigenschaften bei Temperaturen von 20 bis 70 Grad Celsius aufrechterhält.

5. Hydrogel nach Anspruch 1, ferner umfassend mindestens einen Wirkstoff, der ausgewählt ist aus einer Gruppe von kleinen Molekülen, organischen Molekülen, anorganischen Molekülen, kosmetischen Wirkstoffen, pharmakologisch aktiven Verbindungen, Mikroorganismen, Nukleinsäuren, Peptiden oder Proteinen.

6. Hydrogel nach Anspruch 5, wobei der Wirkstoff ein Flammschutzmittel, ein Düngemittel oder ein Pestizid ist.

7. Verwendung des Hydrogels nach Anspruch 5 als ein wässriges Viskositätsmodifizierungsmittel oder zur Verwendung bei der Leitungsinstandhaltung und Produktrückgewinnung.

8. Verwendung des Hydrogels nach Anspruch 6 als Träger für Flammschutzmittel.

9. Verwendung des Hydrogels nach Anspruch 6 als Träger für Pestizide, zur Verwendung als Träger für Düngemittel, zur Verwendung als biomimetisches Gerüst im Gewebe-Engineering oder zur Verwendung bei der Verabreichung von Arzneimitteln und Impfstoffen.

10. Verfahren zur Herstellung des Hydrogels nach Anspruch 1, wobei das Verfahren das Inkontaktbringen einer Vielzahl der kolloidalen Siliziumdioxid-Nanopartikel sowie der HEC und MC in einer solchen Weise umfasst, dass die HEC und MC selektiv an die kolloidalen Siliziumdioxid-Nanopartikel über mehrwertige und nicht-kovalente Wechselwirkungen und Vernetzungen adsorbieren können.

11. Kit zur Bildung des Hydrogels nach Anspruch 1, wobei das Kit eine Vielzahl der kolloidalen Siliziumdioxid-Nanopartikel sowie die HEC und MC umfasst.

12. Verfahren zur Brandverhütung, wobei das Verfahren das Inkontaktbringen von leicht entflammbarem Material mit dem Hydrogel nach Anspruch 6 umfasst.

13. Verfahren zum Molchen von Rohrleitungen, wobei das Verfahren das Inkontaktbringen von Rohrleitungen, die gereinigt oder gespült werden müssen, mit dem Hydrogel nach Anspruch 1 oder 5 umfasst.

14. Verfahren zur kontrollierten Durchführung einer chemischen Behandlung, wobei das Verfahren das Inkontaktbringen von Material, das einer chemischen Behandlung bedarf, mit dem Hydrogel nach Anspruch 5 oder Anspruch 9 umfasst.

## Revendications

1. Hydrogel trimère à réticulation non covalente
comprenant
de l'hydroxyéthylcellulose (HEC),
de la méthylcellulose (MC), et
des nanoparticules de silice colloïdale,
dans lequel la HEC et la MC sont réticulées par les nanoparticules de silice colloïdale pour former l'hydrogel à réticulation non covalente, et dans lequel :
la surface de silice comprend au moins 80 % de groupes silanol et la surface de silice est chargée négativement d'une manière dépendante du pH.

2. Hydrogel selon la revendication 1, dans lequel :
la proportion totale de HEC et de MC est de 0,1 % en poids à 0,2 % en poids de l'hydrogel,
la proportion totale de nanoparticules de silice colloïdale est de 0,5 % en poids à 2 % en poids de l'hydrogel, et
le rapport en poids entre HEC et MC est de 85:15.

3. Hydrogel selon la revendication 1, dans lequel lesdites nanoparticules ont une surface de silice sensiblement constituée de groupes silanol et lesdites nanoparticules sont chargées négativement en fonction de l'alcalinité.

4. Hydrogel selon la revendication 1 qui est capable d'une intensification linéaire tout en restant capable d'une fluidification par cisaillement et d'une auto-réparation et/ou conservant des propriétés mécaniques entre des températures de 20 à 70 degrés Celsius.

5. Hydrogel selon la revendication 1, comprenant en outre au moins un agent sélectionné dans un groupe de petites molécules, de molécules organiques, de molécules inorganiques, d'agents cosmétiques, de composés pharmacologiquement actifs, de micro-organismes, d'acide nucléique, de peptides ou de protéines.

6. Hydrogel selon la revendication 5 dans lequel ledit agent est un agent ignifuge, un engrais ou un pesticide.

7. Utilisation de l'hydrogel de la revendication 5 en tant que modificateur de viscosité aqueux ou pour une utilisation dans l'entretien de canalisations et la valorisation de produits.

8. Utilisation de l'hydrogel de la revendication 6 en tant que support de produits ignifuges.

9. Utilisation de l'hydrogel de la revendication 6 en tant que support de pesticides, pour une utilisation en tant support d'engrais, pour une utilisation en tant qu'échafaudage biomimétique dans l'ingénierie tissulaire, ou pour une utilisation dans l'administration de médicaments et de vaccins.

10. Procédé de préparation de l'hydrogel de la revendication 1, ledit procédé comprenant la mise en contact d'une pluralité desdites nanoparticules de silice colloïdale et desdites HEC et MC de telle sorte que lesdites HEC et MC peuvent s'adsorber sélectivement sur lesdites nanoparticules de silice colloïdale par l'intermédiaire d'une interaction et d'une réticulation multivalentes et non covalentes.

11. Kit destiné à former l'hydrogel de la revendication 1, le kit comprenant une pluralité desdites nanoparticules de silice colloïdale et lesdites HEC et MC.

12. Procédé de prévention des incendies, le procédé comprenant la mise en contact d'un matériau facilement apte à prendre feu avec l'hydrogel de la revendication 6.

13. Procédé de raclage de canalisations, le procédé comprenant la mise en contact de canalisations nécessitant un nettoyage ou une purge avec l'hydrogel de la revendication 1 ou 5.

14. Procédé d'application d'un traitement chimique de manière commandée, le procédé comprenant la mise en contact d'un matériau nécessitant un traitement chimique avec l'hydrogel de la revendication 5 ou de la revendication 9.
